# EUROPEAN PATENT APPLICATION

(11) **EP 4 256 950 A1**
(43) Date of publication of application: **11.10.2023**
(21) Application number: 22305464.4
(22) Date of filing: 06.04.2022
(51) Int. Cl.: A01H 1/00, A01H 5/08, A01H 6/34

(54) **TOLERANCE TO CGMMV IN CUCUMBER**

(71) Applicant: Vilmorin et Cie, 75001 Paris (FR)
(72) Inventor: YOGEV, Ohad, 7983700 TEL AVIV (IL); BUSKILA, Yossi, 7983700 TEL AVIV (IL); PAZ, Zahi, 7983700 TEL AVIV (IL)
(74) Representative: Lavoix

(57) **Abstract**

The present invention relates to resistance and/or tolerance in plants of *Cucumis sativus,* preferably *Cucumis sativus var. sativus*, to tobamovirus, especially to Cucumber Green Mottle Mosaic Virus (CGMMV). The present invention also relates to cucumbers, especially cultivated cucumbers, comprising genetic determinants that lead to tolerance and/or resistance to CGMMV. The invention further related to markers linked to the genetic determinants and to the use of such markers to identify or select the genetic determinants and to identify or select plants carrying such tolerance or resistance. The invention also relates to the seeds and progeny of such plants and to propagation material for obtaining such plants, and to different uses of these plants.

## Description

The present invention relates to resistance and/or tolerance in plants of *Cucumis sativus,* preferably *Cucumis sativus var. sativus,* to tobamovirus, especially to Cucumber Green Mottle Mosaic Virus (CGMMV). The present invention also relates to cucumbers, especially cultivated cucumbers, comprising genetic determinants that lead to tolerance and/or resistance to CGMMV. The invention further related to markers linked to the genetic determinants and to the use of such markers to identify or select the genetic determinants and to identify or select plants carrying such tolerance or resistance. The invention also relates to the seeds and progeny of such plants and to propagation material for obtaining such plants, and to different uses of these plants.

### Background of the invention

Cucumber *(Cucumis sativus* L. 2n = 2x = 14) belongs to the Cucurbitaceae family that includes more than 800 species and is an annual vine plant. Cucumber is one of the most important and widely cultivated vegetables crops, it originates from India, and China is extensively considered as its secondary center of origin. Despite its considerable morphological variability, cucumber showed a narrow genetic base and has a relatively small genome size (350 Mbp), which was sequenced in 2009 (Huang et al., 2009. Nat Genet. 2009 Dec; 41 (12):1275-81). *Cucumis sativus* houses several botanical varieties including *var. sativus,* the cultivated cucumber and the wild, free-living *var. hardwickii* (R.) *Alef.* (Kirkbride, 1993. Biosystematic monograph of the genus Cucumis (Cucurbitaceae). 84).

A variety of pathogens affects the productivity of cucumber plants including viruses, fungi, bacteria, nematodes, and insects. Cucumbers are inter alia susceptible to many viruses and virus resistance is therefore of major agricultural importance.

Viruses in the genus Tobamovirus (family Virgaviridae) include some of the most important plant viruses causing severe losses in many economically important crops. Tobamoviruses infect vegetable crops mostly, solanaceous and cucurbitaceous plants, ornamental plants, weeds and medicinal plants. In the recent years, the spread of Tobamoviruses that infects two major cultivated vegetable crops, the solanaceous and cucurbitaceous plants has increased.

Tobamoviruses constitute a group of plant viruses with single-stranded RNA genome encodes four known proteins: short (126 or 129 kDa) and long (183 or 186 kDa) replicase-associated proteins. The long component is the outcome of a translational read-through of a termination codon of the short component. In addition, a movement protein (MP) of ≈30 kDa and a coat protein (CP) of ≈17 kDa are translated from sub-genomic RNA. A putative fifth 54 kDa protein resides between the two replicase-associated proteins (Smith et al., 2018 Aspect in Tobamovirus Management in intensive Agriculture. Plant Diseases, Chap 3).

Cucumber green mottle mosaic virus (CGMMV) is a tobamovirus causing severe diseases in Cucurbits worldwide. CGMMV is a plus-strand RNA virus species under the genus Tobamovirus forming a distinct evolutionary clade separated from the other cucurbit infecting tobamovirus species. The closest relatives of CGMMV are other cucurbit-infecting tobamoviruses, KGMMV, CFMMV and ZGMMV. In the complete genome sequence, CGMMV shares 59.1-60.4% similarity with the other cucurbit-infecting tobamoviruses and only 46.0-49.3% with the rest of the tobamovirus species (Mandal et al., 2008. Plant Viruses 2(1): 25-34).
Cucumber Green Mottle Mosaic Virus (CGMMV) main hosts are crop species belonging to the Cucurbitaceaen such as, cucumber *(Cucumis sativus),* squash *(Cucurbita moschata)* and watermelon (*Citrullus lanatus*) (Lovisolo, 1981 Acta Horticulturae 88, 33-82).

CGMMV was first reported in cucumber (*Cucumis sativus*) from Great Britain in 1935 by Ainsworth. Its incidence in other countries of the world has increased rapidly during the last decade. Subsequently the virus was reported also from several countries in Asia and Europe e.g., China, Israel, Greece, Japan, India, Korea, Netherlands, Pakistan, Poland, Russia, Saudi Arabia, Spain, Taiwan and Ukraine (Mandal et al., 2008. Plant Viruses 2(1): 25-34).

The typical disease symptoms caused by Cucumber Green Mottle Mosaic Virus (CGMMV) are leaf mottling and mosaic on foliage, plant dwarfing, flesh discoloration, surface mottling, and distortion of fruit. Fruit yield and quality losses can be severe in some cucurbit crop species, including watermelon and cucumber (*Cucumis sativus*) (Fletcher, J.T. et al., 1969. Plant Pathol 18:16-22).

Cucumber Green Mottle Mosaic Virus (CGMMV) is seed-borne, highly contact transmissible. Infection of plants with CGMMV occurs by transmitting through soil and irrigation water contaminated with infected plant debris. Seed transmission of CGMMV has been also described in bottle gourd and cucumber. It has no known insect vector, but recently honey bees (*Apis mellifera*) have been demonstrated to transfer CGMMV during pollination (Darzi et al., 2017. Plant Pathology, 1365-3059). In addition, like other tobamoviruses, CGMMV can survive for a long time on plant debris from infected crops (Reingold et al., 2015. Plant pathology, 64: 245-255).

The international patent application WO2015/150560 disclosed a modified gene located on chromosome 5 called RDR1 conferring an increased resistance to Potyviridae for example the genus Ipomovirus and the genus Potyvirus, and Virgaviridae, which may comprise the genus Tobamovirus.

However, CGMMV is a Tobamovirus, and Tobamoviruses are known to overcome resistances (Luria et al., 2017. PloS One 12(1): e0170429).

Therefore, there is an important need in the art to identify additional reliable sources of resistance and/or tolerance which could be used to obtain resistant commercial cultivated plants of *Cucumis sativus.*

The present invention provides cultivated *C. sativus* plants that display resistance and/or tolerance to Cucumber Green Mottle Mosaic Virus (CGMMV), as well as methods to produce or identify cucumber plants that display resistance and/or tolerance to CGMMV, and potentially also to other Tobamoviruses. The present invention also discloses molecular genetic markers, especially SNPs, linked to the genetic loci conferring resistance and/or tolerance to CGMMV.

### Summary

The present inventors have identified *C. sativus* plants which display a tolerance and/or resistance to the Cucumber Green Mottle Mosaic Virus (CGMMV) and they have been able to localize and identify genetic determinants, also referred hereafter as QTLs (Quantitative Trait Locus) that lead to tolerance and/or resistance to the CGMMV.

The tolerance and/or resistance according to the present invention is imparted by the newly discovered genetic determinants, that can confer a satisfying level of tolerance and/or resistance to the Cucumber Green Mottle Mosaic Virus (CGMMV). The present invention thus provides these genetic determinants, also here named QTLs.

The present invention provides cultivated cucumber plants that display tolerance and/or resistance to CGMMV as well as methods that produce or identify cucumber plants that exhibit tolerance and/or resistance to CGMMV. The present invention also discloses molecular genetic markers, especially SNPs, linked to the QTLs that lead to tolerance and/or resistance to the CGMMV. Plants obtained through the methods and uses of such molecular markers are also provided.

Said resistance and/or tolerance is moreover easily transferable to different genetic backgrounds and the invention also extends to different methods allowing the transfer or introgression of the QTLs conferring the phenotype.

The invention also provides several methods and uses of the information linked to the SNPs associated to the QTLs conferring CGMMV resistance and/or tolerance, inter alia methods for identifying CGMMV resistant and/or tolerant plants and methods for identifying further molecular markers linked to this resistance and/or tolerance, as well as methods for improving the yield of cucumber production in an environment infested by CGMMV and methods for protecting a cucumber field from CGMMV infection or transmission.

### Definitions

The term "Resistance" is as defined by the ISF (International Seed Federation) Vegetable and Ornamental Crops Section for describing the reaction of plants to pests or pathogens, and abiotic stresses for the Vegetable Seed Industry. Specifically, by resistance, it is meant the ability of a plant variety to restrict the growth and development of a specified pest or pathogen and/or the damage they cause when compared to susceptible plant varieties under similar environmental conditions and pest or pathogen pressure. Resistant varieties may exhibit some disease symptoms or damage under heavy pest or pathogen pressure.

The term "Tolerance" is used herein to indicate a phenotype of a plant wherein at least some of the disease-symptoms remain absent upon exposure of said plant to an infective dose of virus, whereby the presence of a systemic or local infection, virus multiplication, at least the presence of viral genomic sequences in cells of said plant and/or genomic integration thereof can be established, at least under some culture conditions. Tolerant plants are therefore resistant for symptom expression but symptomless carriers of the virus. Sometimes, viral sequences may be present or even multiply in plants without causing disease symptoms. It is to be understood that a tolerant plant, although it is infected by the virus, is generally able to restrict at least moderately the growth and development of the virus. Moreover, some plants may be tolerant under some culture conditions, and resistant under different conditions. Tolerance and Resistance are thus not mutually exclusive. Symptoms of CGMMV infection generally include leaf mottling and mosaic on foliage, plant dwarfing, fruity skin discoloration, surface mottling, and distortion of fruit. Susceptibility: The inability of a plant variety to restrict the growth and development of a specified pest or pathogen; a susceptible plant displays the detrimental symptoms linked to the virus infection, namely the foliar damages and fruit damages in case of CGMMV infection.

A "cucumber" denotes a *Cucumis sativus* plant.

A *Cucumis sativus* plant susceptible to CGMMV, is for example the commercially available Romi-F1 (Hazera). All commercially available varieties of cucumbers are, to date, susceptible or do not offer a satisfying level of resistance to CGMMV, before the present invention.

A plant according to the invention has thus at least improved tolerance or resistance to CGMMV, with respect to the variety Romi-F1 (Hazera), and more generally with respect to most of commercial varieties of cucumber.

As used herein, the terms "offspring" or "progeny" refers to any plant resulting as progeny from a vegetative or sexual reproduction from one or more parent plants or descendants thereof. For instance, an offspring plant may be obtained by cloning or selfing of a parent plant or by crossing two parental plants and include selfing as well as the F1 or F2 or still further generations. An F1 is a first-generation offspring produced from parents at least one of which is used for the first time as donor of a trait, while offspring of second generation (F2) or subsequent generations (F3, F4, etc.) are specimens produced from selfing of F1's, F2's etc. An F1 may thus be (and usually is) a hybrid resulting from a cross between two true breeding parents (true-breeding is homozygous for a trait), while an F2 may be (and usually is) an offspring resulting from self-pollination of said F1 hybrids.

As used herein, the terms "cross", "crossing", "cross pollination" or "cross-breeding" refer to the process by which the pollen of one flower on one plant is applied (artificially or naturally) to the ovule (stigma) of a flower on another plant.

As used herein, the terms "genetic determinant" and/or "QTL" refers to any segment of DNA associated with a biological function. Thus, QTLs and/or genetic determinants include, but are not limited to, genes, coding sequences and/or the regulatory sequences required for their expression. QTLs and/or genetic determinants can also include non-expressed DNA segments that, for example, form recognition sequences for other proteins.

As used herein, the terms "molecular marker" or "marker" refer to an indicator that is used in methods for visualizing differences in characteristics of nucleic acid sequences. Examples of such indicators are restriction fragment length polymorphism (RFLP) markers, amplification fragment length polymorphism (AFLP) markers, single nucleotide polymorphisms (SNPs), insertion mutations, microsatellite markers (SSRs), sequence-characterized amplified regions (SCARs), cleaved amplified polymorphic sequence (CAPS) markers or isozyme markers or combinations of the markers described herein which defines a specific genetic and chromosomal location. Mapping of molecular markers in the vicinity of an allele is a procedure which can be performed quite easily by the person skilled in the art using common molecular techniques.

It is noted in this respect that specific positions in a chromosome can indeed be defined with respect to markers, such as SNPs, insofar as the flanking sequences of said markers are defined in order to unambiguously position them on the genome. The present inventors have used SNPs markers, identified by their flanking sequences, present in the cucumber genome, to discriminate between introgressed and endogenously residing sequences and to track down the introgressed sequences conferring the CGMMV resistance in the cucumber genome. Their location in the cucumber genome refer to the v3, Chinese Long 9930", available at http://cucurbitgenomics.org/organism/20 (based on Li et al, 2019 A chromosome-scale genome assembly of cucumber (Cucumis sativus L.). Gigascience, Volume 8, Issue 6, June 2019, giz072). In the sequence associated with a specific SNP in the present application, for example SEQ ID NO:44 for the SNP CU-0002841, only one nucleotide within the sequence corresponds to the polymorphism: namely the 36th nucleotide of SEQ ID NO:44 corresponds to the polymorphic position of SNP CU-0002841, which can be A or G as indicated in table 8. The flanking sequences are given for positioning the SNP in the genome but are not part of the polymorphism as such.

As used herein, a "chromosomal region" or "chromosomal interval" delimited by two markers (e.g. SNPs) X and Y refers to the section of the chromosome lying between the positions of these two markers and comprising said markers; therefore the nucleotide sequence of this chromosomal region or interval begins with the nucleotide corresponding to marker X and ends with the nucleotide corresponding to marker Y, i.e. the markers are comprised within the region or interval they delimit. Thus, these two markers are defining the border of the chromosomal region or interval.

In the sense of the invention, the SNPs defining a border of a QTL (see tables 5, 7 and 9) are used to precisely locate, in the cucumber genome, a chromosomal region comprising a QTL of the invention (i.e., QTL1.2, QTL1.1 or QTL5). Therefore, both allelic versions of these SNPs defining the borders (i.e., tables 5, 7 and 9) can be used to define a border.

Accordingly, a chromosomal region delimited by two SNPs A1 and A2 refers to the section of the chromosome lying between the positions of these two SNPs and comprising said SNPs, therefore the nucleotide sequence of this chromosomal region begins with the nucleotide corresponding to SNP A1 and ends with the nucleotide corresponding to SNP A2, i.e. the SNPs are comprised within the region they delimit, according to the invention.

It is also to be noted that in the tables defining the border of QTL of the invention (i.e., tables 5, 7 and 9) each SNP is classified in a group (X or Y). SNPs from a group X are located at one side of a QTL and SNPs from a group Y are located at the other side of the said QTL. So, each QTL of the invention is located between SNPs from a group X and a group Y. Thus, for each QTL, any SNP from X group can be combined with any SNP from Y group in order to define and delimit the said QTL. For example, the chromosomal region of QTL1.2 can be delimited between CU-0006144 (SEQ ID NO 36, group X) and, CU-0000049 (SEQ ID NO 38, group Y) or CU-0001483 (SEQ ID NO 39, group Y).

As used herein, the term "genotype" refers to the genetic makeup of an individual cell, cell culture, tissue, organism (e.g., a plant), or group of organisms.

As used herein, the term "grafting" is the operation by which a rootstock is grafted with a scion. The primary motive for grafting is to avoid damages by soil-born pest and pathogens when genetic or chemical approaches for disease management are not available. Grafting a susceptible scion onto a resistant rootstock can provide a resistant cultivar without the need to breed the resistance into the cultivar. In addition, grafting may enhance tolerance to abiotic stress, increase yield and result in more efficient water and nutrient uses.

As used herein, the term "heterozygote" refers to a diploid or polyploid individual cell or plant having different alleles (forms of a given gene, genetic determinant or sequences) present at least at one locus. As used herein, the term "heterozygous" refers to the presence of different alleles (forms of a given gene, genetic determinant or sequences) at a particular locus.

As used herein, "homologous chromosomes", or "homologs" (or homologues), refer to a set of one maternal and one paternal chromosome that pair up with each other during meiosis. These copies have the same genes in the same loci and the same centromere location.

As used herein, the term "homozygote" refers to an individual cell or plant having the same alleles at one or more loci on all homologous chromosomes.

As used herein, the term "homozygous" refers to the presence of identical alleles at one or more loci in homologous chromosomal segments.

As used herein, the term "hybrid" refers to any individual cell, tissue or plant resulting from a cross between parents that differ in one or more genes.

As used herein, the term "locus" (plural: "loci") refers to any site that has been defined genetically, this can be a single position (nucleotide) or a chromosomal region. A locus may be a gene, a genetic determinant, or part of a gene, or a DNA sequence, and may be occupied by different sequences. A locus may also be defined by a SNP (Single Nucleotide Polymorphism), by several SNPs, or by two flanking SNPs.

As used herein, the term "rootstock" is the lower part of a plant capable of receiving a scion in a grafting process.

As used herein, the term "scion" is the higher part of a plant capable of being grafted onto a rootstock in a grafting process.

As used herein, the term "commercially acceptable fruit quality", it is meant a fruit of type Long European type, Midi, Beit Alpha, Baby/Mini, Asian, Chinese parth, the fruit can be parthenocarpic or not, 5 to 36 cm long, or/and 1 cm to 7 cm wide, having light green up to dark green or marble skin color, the fruit can be bitter or not. Examples of fruits having a commercially acceptable fruit quality can be the fruits of the ROMI-F1, Carmen or Kingstar varieties.

The invention encompasses plants of different ploidy levels, whether a diploid plant, but also a triploid plant, a tetraploid plant, etc.

In the context of the present invention, DNA strand and allele designation and orientation for the SNP markers is done according to the TOP/BOT method developed by Illumina (https://www.illumina.com/documents/products/technotes/technote_topbot.pdf).

### Detailed description of the invention:

The present inventors have identified a major QTL on chromosome 1 which, when present in *Cucumis sativus* plants, provides to these plants an improved tolerance and/or resistance when infected or likely to be infected by the Cucumber Green Mottle Mosaic Virus (CGMMV). This said QTL is named "QTL1.2".

The plant according to the invention is preferably a cultivated cucumber, namely a cultivated *C. sativus var. sativus* plant.

The phenotype of the plants according to the invention is, in most circumstances, best characterized as tolerance rather than resistance to CGMMV; under specific circumstances, the plants of the invention however exhibit resistance to CGMMV. In the following, reference is made to tolerance to CGMMV; this phenotype however encompasses resistance phenotype, under specific circumstances. Plants of the invention may also behave as symptomless carriers, under certain conditions. Therefore, in the following any reference to tolerance, or tolerance phenotype, can be substituted by a reference to resistance, or by a reference to resistance and/or tolerance.

The tolerance according to the invention is preferably in response to any type of infection, either natural infection or mechanical inoculation, and in any event. It is at least tolerance to natural infection by CGMMV. As describe above, any reference to tolerance can be substituted by a reference to resistance, or to resistance and/or tolerance.

The tolerance according to the invention is preferably characterized by the absence of all the symptoms generally associated with CGMMV infection, especially severe CGMMV, namely leaf mottling and mosaic on foliage, plant dwarfing, flesh discoloration, surface mottling, and distortion of fruit, upon exposure to the virus, in natural infection conditions

According to a first aspect, the present invention is thus directed to a Cucumis Sativus plant tolerant to Cucumber green mottle mosaic virus (CGMMV) comprising in its genome a quantitative trait locus (QTL) on chromosome 1, wherein said QTL confers to the plant a tolerance to CGMMV. The invention is also directed to a cell or seed of such plant, comprising said QTL on chromosome 1 conferring tolerance to CGMMV.

In some embodiments, said QTL on chromosome 1 comprises at leastQTL1.2. Said QTL1.2 can be present at heterozygous state or homozygous state.

The level of CGMMV tolerance imparted by QTL1.2 is however increased if QTL1.2 is homozygous. The invention is thus more specifically directed to a *Cucumis Sativus* plant comprising in its genome QTL1.2 on chromosome 1 conferring a tolerance to CGMMV, wherein said QTL is present homozygously in the genome of said plant, seed or cell thereof, thus imparting a higher tolerance level to CGMMV.

In some embodiments, said QTL1.2 is located on chromosome 1, within the chromosomal region delimited by CU-0006144 (SEQ ID NO:36) and CU-0001483 (SEQ ID NO:39). Preferably, QTL1.2 is located on chromosome 1, within the chromosomal region delimited by CU-0008776 (SEQ ID NO:37) and CU-0000049 (SEQ ID NO:38).

The present inventors have identified that the QTL1.2 present in the genome of the plant, seed or cell of the invention, and which is responsible for the phenotype of interest, i.e. an improved tolerance to CGMMV, is to be found in the chromosomal regions mentioned above, by identifying along said regions the presence of sequences at different loci. The following 14 loci defined by the 14 following SNPs have been identified to be linked to QTL1.2: CU-0006143 (SEQ ID NO:40), CU-0003007 (SEQ ID NO:41), CU-0001935 (SEQ ID NO:42), CU-0003410 (SEQ ID NO:43), CU-0002841 (SEQ ID NO:44), CU-0005159 (SEQ ID NO:45), CU-0003498 (SEQ ID NO:46), CU-0004638 (SEQ ID NO:47) CU-0000697 (SEQ ID NO:48), CU-0000649 (SEQ ID NO:49), CU-0003579 (SEQ ID NO:50), CU-0003759 (SEQ ID NO:51), CU-0001719 (SEQ ID NO:52) and CU-0000987 (SEQ ID NO:53).

The alleles of the SNPs corresponding to the QTL1.2 conferring the CGMMV tolerance are (see table 8): allele A of CU-0006143, allele A of CU-0003007, allele A of CU-0001935, allele A of CU-0003410, allele G of CU-0002841, allele G of CU-0005159, allele A of CU-0003498, allele G of CU-0004638, allele A of CU-0000697, allele G of CU-0000649, allele A of CU-0003579, allele G of CU-0003759, allele G of CU-0001719 and allele A of CU-0000987.

Thus, in some embodiments, said QTL1.2 is detectable by the presence of at least one of the 14 loci described above. Preferred SNPs linked to QTL1.2 are CU-0003410 (SEQ ID NO:43), CU-0002841 (SEQ ID NO:44), CU-0005159 (SEQ ID NO:45), CU-0003498 (SEQ ID NO:46) and CU-0004638 (SEQ ID NO:47). The invention is thus more specifically directed to a *Cucumis Sativus* plant comprising in its genome QTL1.2 on chromosome 1 conferring a tolerance to CGMMV, wherein QTL1.2 comprises at least one of the following SNP markers: CU-0006143 (SEQ ID NO:40), CU-0003007 (SEQ ID NO:41), CU-0001935 (SEQ ID NO:42), CU-0003410 (SEQ ID NO:43), CU-0002841 (SEQ ID NO:44), CU-0005159 (SEQ ID NO:45), CU-0003498 (SEQ ID NO:46), CU-0004638 (SEQ ID NO:47), CU-0000697 (SEQ ID NO:48), CU-0000649 (SEQ ID NO:49), CU-0003579 (SEQ ID NO:50), CU-0003759 (SEQ ID NO:51), CU-0001719 (SEQ ID NO:52) and CU-0000987 (SEQ ID NO:53).

In some embodiments, the *Cucumis Sativus* plant, cell or seed of the invention further comprises another QTL on chromosome 1, namely QTL1.1, and/or a QTL on chromosome 5, namely QTL5, improving the level of CGMMV tolerance. The invention is thus directed to a *Cucumis Sativus* plant comprising in its genome, QTL1.2 and preferably also QTL1.1 and/or QTL5, wherein said QTLs confer to the plant a tolerance to CGMMV.

In some embodiments, said QTL1.1 is present at heterozygous state or homozygous state.

In some embodiments, said QTL5 is present at heterozygous state or homozygous state.

Preferably, the *Cucumis Sativus* plant, cell or seed according to the invention comprises any of the combinations of the QTLs as defined here above. For example, the *Cucumis Sativus* plant, cell or seed according to the invention may comprise the following combinations of the QTLs associated to resistance to CGMMV:
- QTL1.2 and QTL1.1 conferring a tolerance to CGMMV, preferably one or both QTL(s) is(are) present homozygously,
- QTL1.2 and QTL5 conferring a tolerance to CGMMV, preferably one or both QTL(s) is(are) present homozygously, or
- QTL1.2 and QTL1.1 and QTL5 conferring a tolerance to CGMMV, preferably one or two or three QTL(s) is(are) present homozygously

Each QTL conferring tolerance to CGMMV, taken in isolation, provides a moderate but sufficient level of tolerance to CGMMV in case of CGMMV infection, irrespectively of the strain of CGMMV. The combination of at least two QTLs of the invention, as detailed in the above paragraph, confers a higher level of CGMMV tolerance, irrespective of the infecting strain of CGMMV.

Preferably, at least one QTL of the invention (i.e., QTL1.2 or QTL1.1 or QTL5) is homozygously present. More preferably, two QTLs of the invention are present homozygously. Even more preferably, the three QTLs of the invention are present homozygously.

In some embodiments, said QTL1.1 is located on chromosome 1, within the chromosomal region delimited by CU-0001918 (SEQ ID NO:1) and CU-0006297 (SEQ ID NO:8). Preferably, QTL1.1 is located within the chromosomal region delimited by CU-0004565 (SEQ ID NO:2) and CU-0004683 (SEQ ID NO:7), and more preferably located within the chromosomal region delimited by CU-0001726 (SEQ ID NO:3) and CU-0001398 (SEQ ID NO:6), and even more preferably located within the chromosomal region delimited by CU-0001212 (SEQ ID NO:4) and CU-0001683 (SEQ ID NO:5).

In some embodiments, said QTL5 is located on chromosome 5, within the chromosomal region delimited by CU-0005234 (SEQ ID NO: 54) and CU-0007979 (SEQ ID NO: 57). Preferably, QTL5 is located on chromosome 5, within the chromosomal region delimited by CU-0007682 (SEQ ID NO:55) and CU-0000521 (SEQ ID NO:56).

The present inventors have identified that the QTL1.1 and QTL5 present in the genome of the plant, seed or cell of the invention and contributing to the phenotype of interest, i.e. an improved tolerance to CGMMV, are to be found in the chromosomal regions mentioned above, by identifying along said regions the presence of sequences at different loci:
- The following 27 loci defined by the 27 following SNPs have been identified to be linked to QTL1.1: CU-000760 (SEQ ID NO:9), CU-0005918 (SEQ ID NO:10), CU-0001201 (SEQ ID NO:11), CU-0004609 (SEQ ID NO:12), CU-0000028 (SEQ ID NO:13), CU-0003841 (SEQ ID NO:14), CU-0005952 (SEQ ID NO:15), CU-0000131 (SEQ ID NO:16), CU-0004274 (SEQ ID NO:17), CU-0002804 (SEQ ID NO:18), CU-0008736 (SEQ ID NO:19), CU-0000648 (SEQ ID NO:20), CU-0002063 (SEQ ID NO:21), CU-0008746 (SEQ ID NO:22), CU-0002751 (SEQ ID NO:23), CU-0002122 (SEQ ID NO:24), CU-0006155 (SEQ ID NO:25), CU-0001458 (SEQ ID NO:26), CU-0006165 (SEQ ID NO:27), CU-0006164 (SEQ ID NO:28), CU-0006180 (SEQ ID NO:29), CU-0000058 (SEQ ID NO:30), CU-0000848 (SEQ ID NO:31), CU-0001233 (SEQ ID NO:32), CU-0005032 (SEQ ID NO:33), CU-0004710 (SEQ ID NO:34) and CU-0006247 (SEQ ID NO:35);
- The following 7 loci defined by the 7 following SNPs have been identified to be linked to QTL5: CU-0000533 (SEQ ID NO:58), CU-0001575 (SEQ ID NO:59), CU-0009561 (SEQ ID NO:60), CU-0007750 (SEQ ID NO:61), CU-0002973 (SEQ ID NO:62), CU-0001079 (SEQ ID NO:63) and CU-0005413 (SEQ ID NO:64).

The alleles of the SNPs of the invention corresponding to the QTLs conferring the CGMMV tolerance are detailed hereafter (see table 6 and 10):
- The following 27 alleles linked to QTL1.1: allele G of CU-0000760, allele G of CU-0005918, allele G of CU-0001201, allele C of CU-0004609, allele G of CU-0000028, allele A of CU-0003841, allele A of CU-0005952, allele G of CU-0000131, allele G of CU-0004274, allele G of CU-0002804, allele A of CU-0008736, allele G of CU-0000648, allele A of CU-0002063, allele G of CU-0008746, allele A of CU-0002751, allele A of CU-0002122, allele C of CU-0006155, allele G of CU-0001458, allele G of CU-0006165, allele G of CU-0006164, allele A of CU-0006180, allele A of CU-0000058, allele C of CU-0000848, allele G of CU-0001233, allele C of CU-0005032, allele A of CU-0004710 and allele A of CU-0006247;
- The following 7 alleles linked to QTL5: allele G of CU-0000533, allele G of CU-0001575, allele A of CU-0009561, allele G of CU-0007750, allele A of CU-0002973, allele G of CU-0001079 and allele A CU-0005413.

The presence of the QTL(s) conferring the tolerance to CGMMV can be revealed by the presence of said specific alleles. The alleles of these SNPs can thus reflect the presence of the QTL(s) of the invention. At least one SNP is to be checked for reflecting the presence of the QTL1.1 on chromosome 1. At least one SNP is to be checked for reflecting the presence of the QTL1.2 on chromosome 1. At least one SNP is to be checked for reflecting the presence of QTL5 on chromosome 5.

The invention is thus more specifically directed to plants comprising in their genome QTL1.2, wherein said QTL confers a tolerance to CGMMV, and said QTL1.2 is detectable by the presence of at least one of the 14 loci described above. Preferably said plants further comprise at least one additional QTL, namely QTL1.1 and/or QTL5. QTL1.1 is detectable by the presence of at least one of the 27 loci described above. QTL5, detectable by the presence of at least one of the 7 loci described above. Preferred SNPs linked to QTL1.1 are CU-0000131 (SEQ ID NO:16), CU-0004274 (SEQ ID NO:17), CU-0002804 (SEQ ID NO:18), CU-0008736 (SEQ ID NO:19) and CU-0000648 (SEQ ID NO:20), and preferred SNPs linked to QTL1.2 are CU-0003410 (SEQ ID NO:43), CU-0002841 (SEQ ID NO:44), CU-0005159 (SEQ ID NO:45), CU-0003498 (SEQ ID NO:46) and CU-0004638 (SEQ ID NO:47), and preferred SNPs linked to QTL5 are CU-0009561 (SEQ ID NO:60), CU-0007750 (SEQ ID NO:61) and CU-0002973 (SEQ ID NO:62).

According to another aspect, the invention is also directed to a plant, or cell or seed thereof, comprising in its genome QTL1.1 or/and QTL1.2 on chromosome 1 conferring a tolerance to CGMMV, wherein at least one of the said QTLs is present homozygously in the genome of said plant, seed or cell, thus imparting a higher tolerance level to CGMMV. Preferably, said plant, seed or cell, further comprises in its genome QTL5 in combination with QTL1.1 and/or QTL1.2. More preferably, said QTL5 is homozygously present.

The invention is also directed to hybrid plants of *C. sativus,* obtainable by crossing a *C. sativus* plant having the improved phenotype and bearing QTL1.2, and optionally bearing QTL1.1 and/or QTL5, with another C. *sativus* plant. Preferably said *C. sativus* plant having an improved phenotype is bearing the three QTLs of the invention homozygously. If the other C. *sativus* crossing partner is devoid of the QTL of the present invention, the hybrid resulting from the cross will harbor the QTL of the present invention in a heterozygous state and the cucumber plants will also have the tolerance phenotype, although the level of CGMMV tolerance will be intermediate.

Preferably, a *C. sativus* plant according to the invention is a cultivated plant or line, more preferably a commercial plant or line or hybrid. Such a plant or line thus has generally 4 primary branches or less, generally less than 3 and more preferably has a single primary branch.

Such a commercial plant or line preferably also exhibits resistance to other viruses, especially to potyviruses. Preferably, a commercial plant is resistant to ZYMV (Zucchini Yellow Mosaic Virus) and/or to CVYV (Cucumber Vein Yellowing Virus). Resistances to PRSV (Papaya Ringspot Virus) and WMV (Watermelon Mosaic Virus) and CMV (Cucumber Mosaic Virus) are also generally found in commercial plants. A plant of the invention is thus advantageously resistant at least to powdery mildew and to potyviruses.

Other resistances or tolerances are also envisaged according to the invention, inter alia resistance to downy mildew caused *Pseudoperonospora cubensis,* resistance to Fusaria caused by *Fusariumoxysporum f.sp. cucumerinum* or by *Fusarium oxysporum f.sp. radicis cucumerinum,* resistance to Scab caused by *Cladosporium cu*cumerinum, resistance to WMV, to CYSDV and to CCYV.

Advantageously, a plant of the invention is also resistant to one or more arthropod pests, such as Western flower Thrips (*Franklineilla occidentalis*), Silver leaf white fly (*Bemisia tabaci*), Glasshouse whitefly (*Trialeurodes vaporariorum*) and Root-knot nematode (*Meloidogyne incognita).*

In some embodiments, a plant of the invention is used as a scion or as a rootstock in a grafting process. Grafting is a process that has been used for many years in crops such as cucurbitacea. Grafting may be used to provide a certain level of resistance to telluric pathogens such as Phytophthora or to certain nematodes. Grating is therefore intended to prevent contact between the plant or variety to be cultivated and the infested soil. The variety of interest used as the graft or scion, optionally an F1 hybrid, is grafted onto the tolerant plant used as the rootstock. The tolerant rootstock remains healthy and provides, from the soils, the normal supply for the graft that it isolates from the diseases.

A plant according to the invention is preferably a gynoecious plant, or a monoecious plant, with at least 50% of gynoecious flowers, even more preferably at least 70 or 80% gynoecious flowers. Moreover, a commercial plant of the invention gives rise to fruits in suitable conditions, 50% of which are at least more than 10 cm long at full maturity, preferably more than 15 or 20 cm at full maturity. The harvested fruits have a green skin color, they preferably have an elongated form.

As detailed above, the invention is directed to *C. sativus* plants, exhibiting the CGMMV tolerance, as well as to seeds giving rise to those plants, and cells of these plants or seeds, comprising the tolerance QTL(s).

The invention encompasses plants, seeds and cells of any ploidy levels; it encompasses inter alia diploid, triploid, tetraploid and/or allopolyploid plants, cells or seeds.

A plant or seed according to the invention may be a progeny or offspring of a plant grown from the deposited seeds CUC58.4, deposited at the NCIMB under the accession number NCIMB 43844. Plants grown from the deposited seeds are indeed homozygous for the QTLs of the invention conferring the improved phenotype, they thus bear in their genome the three QTLs (QTL1.1, QTL1.2 and QTL5) of interest on each of the homologues of chromosomes 1 and 5. They can be used to transfer these sequences into another background by crossing and selfing and/or backcrossing.

The invention is also directed to the deposited seeds of CUC58.4 (NCIMB 43844) and to plants grown from one of these seeds. These seeds contain homozygously the QTLs conferring the phenotype of interest. It is noted that these seeds do not correspond to a plant variety, they are not homozygous for most of the genes except the QTLs of the invention; their phenotype is thus not fixed during propagation, except for the tolerance of the invention; most of their phenotypic traits segregate during propagation, with the exception of CGMMV tolerance of the invention. The invention is also directed to plants or seeds as defined above, i.e. containing the three QTLs of interest in homozygous or heterozygous state, but most preferably at least one of those three in homozygous state, said QTLs conferring the CGMMV tolerance, wherein these plants or seeds are obtainable by transferring the QTLs from a *C. sativus* plant, representative seeds thereof were deposited under NCIMB accession NCIMB 43844, into another *C. sativus* genetic background, for example by crossing said deposited plant with a second cucumber plant parent and selection of the plant bearing the QTLs responsible for the phenotype of interest. During such crossing, QTL1.1 and QTL1.2 and QTL5 can be transferred.

The QTLs according to the invention and conferring the improved tolerance to CGMMV are preferably chosen from the ones present in the genome of seeds of CUC58.4. A sample of this C. *sativus var. sativus* seed has been deposited by Vilmorin & Cie, 4 quai de la Mégisserie 75001 Paris, FRANCE, pursuant to and in satisfaction of the requirements of the Budapest treaty on the International Recognition of the deposit of Microorganisms for the Purpose of Patent procedure ("the Budapest Treaty" with the National collection of Industrial, Food and Marine bacteria (NCIMB) (NCIMB, Ltd, Ferguson Building, Craibstone Estate, Bucksburn, Aberdeen AB21 9YA, united Kingdom) on August 16th, 2021 under accession number NCIMB 43844.

It is noted that the seeds or plants of the invention may be obtained by different processes and are not exclusively obtained by means of an essentially biological process.

According to such an aspect, the invention relates to a cucumber plant or seed, preferably a non-naturally occurring cucumber plant or seed such as a modified cucumber plant or seed, which comprises one or more mutations in its genome, which provides the plant the resistance to CGMMV, which mutation is as present, for example, in the genome of plants of which a representative sample was deposited with the NCIMB under deposit number NCIMB 43844.

In some embodiments, the invention relates to a method for obtaining a modified cucumber plant or seed carrying one or more mutations in its genome, which provides the plant the resistance to CGMMV. Such a method may comprise:
a) treating M0 seeds of a CGMMV plant to be modified with a mutagenic agent to obtain M1 seeds;
b) growing plants from the thus obtained M1 seeds to obtain M1 plants;
c) producing M2 seeds by self-fertilisation of M1 plants; and
d) optionally repeating step b) and c) n times to obtain M1+n seeds.

The M1+n seeds are grown into plants and submitted to CGMMV infection. The surviving plants, or those with the less symptoms of CGMMV infection, are multiplied one or more further generations while continuing to be selected for their resistance to CGMMV.

In this method, the M1 seeds of step a) can be obtained via chemical mutagenesis such as EMS mutagenesis. Other chemical mutagenic agents include but are not limited to, diethyl sufate (des), ethyleneimine (ei), propane sultone, N-methyl-N-nitrosourethane (mnu), N-nitroso-N-methylurea (NMU), N-ethyl-N-nitrosourea(enu), and sodium azide.

Alternatively, the mutations are induced by means of irradiation, which is for example selected from x-rays, fast neutrons, UV radiation.

In some embodiments of the invention, the mutations are induced by means of genetic engineering. Such mutations include the integration of sequences conferring the CGMMV resistance, as well as the substitution of residing sequences by alternative sequences conferring the CGMMV resistance or tolerance. Preferably, the mutations are the integration of QTL1.2 with optionally QTL1.1 and/or QTL5, as described above, in replacement of the homologous sequences of a Cucumber plant. More preferably, the mutation is the substitution of the sequence comprised within SNP CU-0006144 (SEQ ID NO:36) and SNP CU-0001483 (SEQ ID NO 39) on chromosome 1 of Cucumber genome, or a fragment thereof, by the homologous sequence on chromosome 1 present in the genome of a plant of which a representative sample was deposited with the NCIMB under deposit number NCIMB 43844, wherein the sequence or fragment thereof confers resistance to CGMMV. Optionally, an additional mutation is a substitution of the sequence comprised within SNP CU-0005234 (SEQ ID NO:54) and CU-0007979 (SEQ ID NO:57) on chromosome 5 of CGMMV genome, or a fragment thereof, by the homologous sequence on chromosome 5, and/or, an additional mutation is a substitution of the sequence comprised within SNP CU-0001918 (SEQ ID NO:1) and SNP CU-0006292 (SEQ ID NO 8) on chromosome 1 of Cucumber genome, or a fragment thereof, by the homologous sequence on chromosome 1 present in the genome of a plant of which a representative sample was deposited with the NCIMB under deposit number NCIMB 43844, wherein the sequence or fragment thereof confers resistance to CGMMV.

The genetic engineering means which can be used include the use of all such techniques called New Breeding Techniques which are various new technologies developed and/or used to create new characteristics in plants through genetic variation, the aim being targeted mutagenesis, targeted introduction of new genes or gene silencing (RdDM). Example of such new breeding techniques are targeted sequence changes facilitated through the use of Zinc finger nuclease (ZFN) technology (ZFN-1, ZFN-2 and ZFN-3, see U.S. Pat. No. 9,145,565), Oligonucleotide directed mutagenesis (ODM), Cisgenesis and intragenesis, Grafting (on GM rootstock), Reverse breeding, Agro-infiltration (agro-infiltration "sensu stricto", agro-inoculation, floral dip), Transcription Activator-Like Effector Nucleases (TALENs, see U.S. Pat. Nos. 8,586,363 and 9,181,535), the CRISPR/Cas system (see U.S. Pat. Nos. 8,697,359; 8,771,945; 8,795,965; 8,865,406; 8,871,445; 8,889,356; 8,895,308; 8,906,616; 8,932,814; 8,945,839; 8,993,233; and 8,999,641), engineered meganuclease re-engineered homing endonucleases, DNA guided genome editing (Gao et al., Nature Biotechnology (2016)), and Synthetic genomics. A major part of targeted genome editing, another designation for New Breeding Techniques, is the applications to induce a DNA double strand break (DSB) at a selected location in the genome where the modification is intended. Directed repair of the DSB allows for targeted genome editing. Such applications can be utilized to generate mutations (e.g., targeted mutations or precise native gene editing) as well as precise insertion of genes (e.g., cisgenes, intragenes, or transgenes). The applications leading to mutations are often identified as site-directed nuclease (SDN) technology, such as SDN1, SDN2 and SDN3. For SDN1, the outcome is a targeted, non-specific genetic deletion mutation: the position of the DNA DSB is precisely selected, but the DNA repair by the host cell is random and results in small nucleotide deletions, additions or substitutions. For SDN2, a SDN is used to generate a targeted DSB and a DNA repair template (a short DNA sequence identical to the targeted DSB DNA sequence except for one or a few nucleotide changes) is used to repair the DSB: this results in a targeted and predetermined point mutation in the desired gene of interest. As to the SDN3, the SDN is used along with a DNA repair template that contains new DNA sequence (e.g. gene). The outcome of the technology would be the integration of that DNA sequence into the plant genome. The most likely application illustrating the use of SDN3 would be the insertion of cisgenic, intragenic, or transgenic expression cassettes at a selected genome location. A complete description of each of these techniques can be found in the report made by the Joint Research Center (JRC) Institute for Prospective Technological Studies of the European Commission in 2011 and titled "New plant breeding techniques - State-of-the-art and prospects for commercial development".

According to such an aspect, the invention relates to a modified cultivated cucumber plant or seed, preferably a non-naturally occurring cucumber plant or seed, which may comprise one or more mutations in its genome, which provides the plant with tolerance to CGMMV, which mutation is as present, for example, in the genome of plants of which a representative sample was deposited with the NCIMB under deposit number NCIMB 43844.

In another embodiment, the invention relates to a method for obtaining a modified cucumber plant or seed carrying one or more mutations in its genome, which provides the plant with tolerance to CGMMV.

The invention in another aspect also concerns any plant likely to be obtained from seed or plant of the invention, and also plant parts of such a plant. Preferably explant, scion, cutting, seed, fruit, root, rootstock, pollen, ovule, embryo, protoplast, leaf, anther, stem, petiole, and any other plants part, wherein said plant, explant, scion, cutting, seed, fruit, root, rootstock, pollen, ovule, embryo, protoplast, leaf, anther, stem, petiole, and/or plant part is obtainable from a seed or plant according to the first aspect of the invention, i.e. bearing the QTL1.2, and optionally QTL1.1 and/or QTL5, of the present invention, homozygously or heterozygously in their genome, wherein preferably at least one QTL is present homozygously. These plant parts, inter alia explant, scion, cutting, seed, fruit, root, rootstock, pollen, ovule, embryo, protoplast, leaf, anther, stem or petiole, comprise in their genome said QTL(s), conferring the phenotype of interest, i.e. tolerance to CGMMV; they thus comprise a cell comprising the QTL(s).

According to a preferred embodiment, the invention is directed to a seed which is capable of developing into a plant according to the invention, thus tolerant to CGMMV infection. The QTL1.2, and optionally QTL1.1 and/or QTL5, referred to in this aspect of the invention are those defined above in the context of plants of the invention. The different features of the QTLs defined in relation with the plant of the invention apply mutatis mutandis to this aspect of the invention. The QTLs are thus preferably chosen from those present in the genome of a plant corresponding to the deposited material CUC58.4 (NCIMB accession number 43844). Each QTL is advantageously characterized by the presence of the following alleles:
- QTL1.2 is preferably characterized by the presence of at least one of the following 14 alleles: allele A of CU-0006143, allele A of CU-0003007, allele A of CU-0001935, allele A of CU-0003410, allele G of CU-0002841, allele G of CU-0005159, allele A of CU-0003498, allele G of CU-0004638, allele A of CU-0000697, allele G of CU-0000649, allele A of CU-0003579, allele G of CU-0003759, allele G of CU-0001719 and allele A of CU-0000987;
- QTL1.1 is preferably characterized by the presence of at least one of the following 27 alleles: allele G of CU-0000760, allele G of CU-0005918, allele G of CU-0001201, allele C of CU-0004609, allele G of CU-0000028, allele A of CU-0003841, allele A of CU-0005952, allele G of CU-0000131, allele G of CU-0004274, allele G of CU-0002804, allele A of CU-0008736, allele G of CU-0000648, allele A of CU-0002063, allele G of CU-0008746, allele A of CU-0002751 , allele A of CU-0002122, allele C of CU-0006155, allele G of CU-0001458, allele G of CU-0006165, allele G of CU-0006164, allele A of CU-0006180, allele A of CU-0000058, allele C of CU-0000848, allele G of CU-0001233, allele C of CU-0005032, allele A of CU-0004710 and allele A of CU-0006247;
- QTL5 is preferably characterized by the presence of at least one of the following 7 alleles: allele G of CU-0000533, allele G of CU-0001575, allele A of CU-0009561, allele G of CU-0007750, allele A of CU-0002973, allele G of CU-0001079 and allele A CU-0005413.

The invention is also directed to cells of C. sativus plants, such that these cells comprise in their genome, the QTL1.2, and optionally QTL1.1 and/or QTL5, of the present invention conferring CGMMV tolerance to a *C. sativus* plant. The QTL1.2, and optionally QTL1.1 and/or QTL5, are those already defined in the context of the present invention, they are characterized by the same features and preferred embodiments already disclosed with respect to the plants and seeds according to the preceding aspects of the invention. The presence of the QTL(s) can be revealed by the techniques disclosed above and well known to the skilled reader. It can inter alia be determined whether the QTL is present homozygously or heterozygously in the genome of such a cell of the invention. Cells according to the invention can be any type of C. *sativus* cell, inter alia an isolated cell and/or a cell capable of regenerating a whole *C. sativus* plant, bearing the QTL(s) of the invention.

In one embodiment, the cell described above is an isolated cell.

The present invention is also directed to a tissue culture of non-regenerable or regenerable cells of the plant according to the present invention. The non-regenerable or regenerable cells comprise in their genome the QTL(s) of the invention conferring CGMMV tolerance, in particular QTL1.2, and optionally, QTL1.1 and/or QTL5, Preferably at least one QTL is present homozygously. Preferably, the regenerable cells are derived from embryos, protoplasts, meristematic cells, callus, pollen, leaves, anthers, stems, petioles, roots, root tips, fruits, seeds, flowers, cotyledons, and/or hypocotyls of the invention, and the cells contain the QTL(s) of the invention in their genome conferring CGMMV tolerance, especially when at least one QTL is present homozygously.

The tissue culture will preferably be capable of regenerating plants having the physiological and morphological characteristics of the foregoing cucumber plant, and of regenerating plants having substantially the same genotype as the foregoing cucumber plant. The present invention also provides cucumber plants regenerated from the tissue cultures of the invention.

The invention also provides a protoplast of the plant defined above, or from the tissue culture defined above, said protoplast containing the QTL(s) conferring the improved phenotype of the invention.

According to another aspect, the present invention is also directed to the use of a cucumber plant of the invention, comprising QTL1.2, and optionally further comprising QTL1.1 and/or QTL5, as a breeding partner in a breeding program for obtaining *C. sativus* plants having CGMMV tolerance. Indeed, such a breeding partner harbors, preferably homozygously in its genome at least one QTL of the invention conferring the tolerance of interest. By crossing this plant with a cucumber plant, especially a line, it is thus possible to transfer the QTL(s) of the present invention conferring the desired phenotype, to the progeny. A plant according to the invention can thus be used as a breeding partner for introgressing the QTL(s) conferring the desired CGMMV tolerance into a *C. sativus* plant or germplasm. Although a plant or seed bearing the QTL(s) of interest heterozygously, can also be used as a breeding partner as detailed above, the segregation of the phenotype is likely to render the breeding program more complex.

The improved phenotype of the invention is tolerance to CGMMV.

The introgressed QTL(s) will advantageously be introduced into varieties that contain other desirable genetic traits such as resistance to other diseases or pest, early fruit maturation, drought tolerance, fruit shape, and the like.

In some embodiments, the plants used are plants or seeds of CUC58.4, deposited at the NCIMB under the accession number NCIMB 43844. Said plants are also suitable as introgression partners in a breeding program aiming at conferring the desired phenotype to a *C. sativus* plant or germplasm.

In such a breeding program, the selection of the progeny displaying the desired phenotype, or bearing the QTL(s) linked to CGMMV tolerance can advantageously be carried out on the basis of the alleles of the SNP markers, especially the SNP markers of the invention.

According to a preferred embodiment, a progeny of the plant is selected on the presence of:
- at least one allele linked to QTL1.2: allele A of CU-0006143, allele A of CU-0003007, allele A of CU-0001935, allele A of CU-0003410, allele G of CU-0002841, allele G of CU-0005159, allele A of CU-0003498, allele G of CU-0004638, allele A of CU-0000697, allele G of CU-0000649, allele A of CU-0003579, allele G of CU-0003759, allele G of CU-0001719 and allele A of CU-0000987, and
- Optionally at least one allele linked to QTL1.1: allele G of CU-0000760, allele G of CU-0005918, allele G of CU-0001201, allele C of CU-0004609, allele G of CU-0000028, allele A of CU-0003841, allele A of CU-0005952, allele G of CU-0000131, allele G of CU-0004274, allele G of CU-0002804, allele A of CU-0008736, allele G of CU-0000648, allele A of CU-0002063, allele G of CU-0008746, allele A of CU-0002751, allele A of CU-0002122, allele C of CU-0006155, allele G of CU-0001458, allele G of CU-0006165, allele G of CU-0006164, allele A of CU-0006180, allele A of CU-0000058, allele C of CU-0000848, allele G of CU-0001233, allele C of CU-0005032, allele A of CU-0004710 and allele A of CU-0006247, and/or
- Optionally at least one allele linked to QTL5: allele G of CU-0000533, allele G of CU-0001575, allele A of CU-0009561, allele G of CU-0007750, allele A of CU-0002973, allele G of CU-0001079 and allele A CU-0005413.

The progeny of the plant is preferably selected on the presence of the same allele on both homologues of each chromosome, for at least one of the SNPs, which is thus present homozygously for a diploid plant.

The selection of the progeny is preferably made on the basis of three SNPs or more, at least one SNP allele for QTL1.1, at least one SNP allele for QTL1.2 and at least one SNP allele for QTL5, wherein at least one of the SNPs is homozygous.

Such a selection will be made on the presence of the alleles of interest in a genetic material sample of the plant to be selected. The presence of these alleles indeed confirms the presence of a QTL(s) of the invention at the loci defined by said SNPs. Moreover, further to point mutation or recombination event, it is conceivable that at least 1 or 2 of these alleles is lost, the remaining of the chromosomal fragment bearing the QTL(s) of interest still conferring the phenotype of interest.

A plant according to the invention or grown from a seed as deposited under accession number NCIMB 43844, is thus particularly valuable in a marker assisted selection for obtaining commercial cucumber lines and varieties, having the improved phenotype of the invention, namely CGMMV tolerance.

The invention is also directed to the use of said plant in a program aiming at identifying, sequencing and / or cloning the genetic sequences conferring the desired phenotype.

Any specific embodiment described for the previous aspects of the invention is also applicable to this aspect of the invention, especially with regard to the features of the QTLs conferring the phenotype of interest.

According to a still another aspect, the invention also concerns methods or processes for the production of *C. sativus* plants, especially C. *sativus var sativus,* inter alia cultivated cucumbers, having the desired phenotype, especially commercial plants and inbred parental lines. The present invention is indeed also directed to transferring the QTL(s) of the invention, QTL1.2, and optionally QTL1.1 and/or QTL5, conferring the CGMMV tolerance, and preferably at least one being homozygous, to other cucumber varieties, or other species or inbred parental lines, and is useful for producing new types and varieties of cucumber.

A method or process for the production of a plant having these features may comprise the following steps:
a) Crossing a plant grown from a plant bearing QTL1.2, and optionally bearing QTL1.1 and/or QTL5, preferably at least one of the said QTL(s) being homozygously present, conferring CGMMV tolerance, and an initial *C. sativus* plant, preferably devoid of said QTLs,
b) Selecting one plant in the progeny thus obtained, bearing the QTL1.2, and optionally QTL1.1 and/or QTL5 of the present invention,
c) Optionally self-pollinating one or several times the plant obtained at step b) and selecting in the progeny thus obtained a plant having tolerance to CGMMV.

Alternatively, the method or process may comprise instead of step a) the following steps:
a1) Crossing a plant grown from a plant bearing QTL1.2, and optionally bearing QTL1.1 and QTL5, or progeny thereof conferring CGMMV tolerance, and an initial C. *sativus* plant, preferably devoid of said QTLs,
a2) Increasing the F1 hybrid by means of selfing to create F2 population.

In the above methods or processes, SNPs markers are preferably used in steps b) and/or c), for selecting plants bearing sequences conferring the tolerance phenotype of interest.

The SNP markers used are preferably the markers of the invention, including all combinations thereof as mentioned elsewhere in the present application, especially:
- At least one SNP linked to QTL1.2, and
- Optionally at least one SNP linked to QTL1.1, and/or at least one SNP linked to QTL5.

By selecting a plant on the basis of the allele of one or more SNPs, it is to be understood that the plant is selected as having tolerance to the CGMMV, or as having QTL1.2, and optionally QTL1.1 and/or QTL5, with respect to the initial plant, when the allele of the SNP is the allele corresponding to the allele of the tolerant parent for this SNP and not the allele of the initial *C. sativus* plant. Those alleles for the 48 SNPs of the invention have already been detailed in this application.

Preferably, the *C. sativus* plant of step a) is an elite line, used in order to obtain a plant with commercially desired traits or desired horticultural traits.

A method or process as defined above may advantageously comprise backcrossing steps, preferably after step c), in order to obtain plants having all the characterizing features of *C. sativus* plants, especially *C. sativus. var sativus.* Consequently, a method or process for the production of a plant having these features may also comprise the following additional steps:
d) Backcrossing the tolerant plant selected in step b) or c) with a *C. sativus* plant;
e) Selecting a plant bearing QTL1.2, and optionally QTL1.1 and/or QTL5 of the present invention.

The plant used in step a), namely any plant according to the 1st aspect of the invention; it may preferably be a plant grown from the deposited seeds, bearing homozygously the three QTLs of the invention conferring the phenotype of interest.

At step e), SNPs markers can be used for selecting plants having an improved tolerance to CGMMV, with respect to the initial plant. The SNP markers are those of the invention, as described in the previous sections.

According to a preferred embodiment, the method or process of the invention is carried out such that, for at least one of the selection steps, namely b), c) and/or e), the selection is based on the detection of at least one of the tolerance alleles reported in tables 6, 8 and 10.

It is to be noted that, when plants having the improved phenotype, and bearing QTL1.2 with optionally QTL1.1 and/or QTL5, the selection is to be made on the basis of one or more the SNPs of the invention, on the presence of the allele(s) representative of the QTL(s), namely the alleles found in the tolerant parent.

The plant selected at step e) is preferably a cultivated or commercial plant, especially a plant having marketable fruit in normal culture conditions, e.g. 50% of the fruits are at least more than 10 cm long at full maturity, preferably more than 15 or 20 cm at full maturity. The harvested fruits have a green skin color, they preferably have an elongated form.

Preferably, steps d) and e) are repeated at least twice and preferably three times, not necessarily with the same *C. sativus* plant. Said *C. sativus* plant is preferably a breeding line.

Resistance to potyviruses and/or powdery mildew may additionally be selected, at each selection step of the processes disclosed above.

Additionally, the selection may also take account of the proportion of gynoecious flowers, wherein gynoecious plants, or monoecious plants having at least 50%, preferably at least 70% or 80% gynoecious flowers, are selected.

It is also preferred that the selected plant has a number of primary branches which is 4 or less than 4, more preferably 3 or less, e.g. 2, most preferably a plant having only one primary branch.

The self-pollination and backcrossing steps may be carried out in any order and can be intercalated, for example a backcross can be carried out before and after one or several self-pollinations, and self- pollinations can be envisaged before and after one or several backcrosses.

The selection of the progeny having the desired improved phenotype can also be made on the basis of the comparison of the CGMMV tolerance from the *C. sativus* parent, through protocols as disclosed inter alia in the examples.

The method used for allele detection can be based on any technique allowing the distinction between two different alleles of a SNP, on a specific chromosome.

The present invention also concerns a plant obtained or obtainable by such a method. Such a plant is indeed a C. sativus plant having the improved phenotype according to the first aspect of the invention.

The invention is also directed to a method for obtaining cultivated or commercial cucumber plants or inbred parental lines thereof, having the desired improved phenotype, corresponding to an improved tolerance to the CGMMV with respect to an initial commercial *C. sativus* plant, comprising the steps of:
a) Backcrossing a plant bearing QTL1.2, and optionally QTL1.1 and/or QTL5 conferring CGMMV tolerance, with a commercial *C. sativus* plant,
b) Selecting a plant bearing QTL1.2, and optionally QTL1.1 and/or QTL5.

Preferably plant of step a) are obtained by germinating a deposited seed CUC58.4 NCIMB accession number 43844, or progeny thereof, bearing QTL1.1, QTL1.2 and QTL5

Preferably, the selection is made based on the presence of at least one of the 14 SNPs linked to QTL1.2, and optionally, (i) at least one of the 27 SNPs linked to QTL1.1, and/or (ii) at least one of the 7 SNPs linked to QTL5. According to a preferred embodiment, the progeny bearing QTL1.2 and optionally QTL1.1 and/or QTL5 comprises at least one of them at the homozygous state.

The present invention is also directed to a *C. sativus* plant and seed obtainable by any of the methods and processes disclosed above. The seed of such *C. sativus* are preferably coated or pelleted with individual or combined active species such as plant nutrients, enhancing microorganisms, or products for disinfecting the environment of the seeds and plants. Such species and chemicals may be a product that promotes the growth of plants, for example hormones, or that increases their resistance to environmental stresses, for example defense stimulators, or that stabilizes the pH of the substrate and its immediate surroundings, or alternatively a nutrient.

They may also be a product for protecting against agents that are unfavorable toward the growth of young plants, including herein viruses and pathogenic microorganisms, for example a fungicidal, bactericidal, hematicidal, insecticidal or herbicidal product, which acts by contact, ingestion or gaseous diffusion; it is, for example, any suitable essential oil, for example extract of thyme. All these products reinforce the resistance reactions of the plant, and/or disinfect or regulate the environment of said plant. They may also be a live biological material, for example a nonpathogenic microorganism, for example at least one fungus, or a bacterium, or a virus, if necessary with a medium ensuring its viability; and this microorganism, for example of the pseudomonas, bacillus, trichoderma, clonostachys, fusarium, rhizoctonia, etc. type stimulates the growth of the plant, or protects it against pathogens.

In all the previous methods and processes, the identification of the plants bearing the QTL(s) responsible for the improved tolerance to the CGMMV could be done by the detection of at least one of the alleles linked with each of the QTL(s), but also in combination with the absence of the other allelic form of the SNPs of the present invention, in order to confirm the homozygous state of at least one of the QTL. As such, the identification of a plant bearing homozygously QTL1.2 of the present invention will be based on the identification of allele A of CU-0006143, allele A of CU-0003007, allele A of CU-0001935, allele A of CU-0003410, allele G of CU-0002841, allele G of CU-0005159, allele A of CU-0003498, allele G of CU-0004638, allele A of CU-0000697, allele G of CU-0000649, allele A of CU-0003579, allele G of CU-0003759, allele G of CU-0001719 and allele A of CU-0000987 as well as the absence of the other allele for these SNPs. Similarly, the identification of a plant bearing homozygously QTL1.1 of the present invention will be based on the identification of allele ; allele G of CU-0000760, allele G of CU-0005918, allele G of CU-0001201, allele C of CU-0004609, allele G of CU-0000028, allele A of CU-0003841, allele A of CU-0005952, allele G of CU-0000131, allele G of CU-0004274, allele G of CU-0002804, allele A of CU-0008736, allele G of CU-0000648, allele A of CU-0002063, allele G of CU-0008746, allele A of CU-0002751 , allele A of CU-0002122, allele C of CU-0006155, allele G of CU-0001458, allele G of CU-0006165, allele G of CU-0006164, allele A of CU-0006180, allele A of CU-0000058, allele C of CU-0000848, allele G of CU-0001233, allele C of CU-0005032, allele A of CU-0004710 and allele A of CU-0006247, as well as the absence of the other allele for these SNPs. And the identification of a plant bearing homozygously QTL5 of the present invention will be based on the identification of allele G of CU-0000533, allele G of CU-0001575, allele A of CU-0009561, allele G of CU-0007750, allele A of CU-0002973, allele G of CU-0001079 and allele A of CU-0005413 as well as the absence of the other allele for these SNPs.

The invention is also directed to the use of the information provided herewith by the present inventors, namely the existence of 3 QTLs, present in the deposited seeds of CUC58.4 (NCIMB accession number 43844), and conferring the improved phenotype to C. *sativus* plants and the disclosure of molecular markers associated to these QTLs. This knowledge can be used inter alia for precisely mapping the QTL(s), for defining their sequences, for identifying cucumber plants comprising the QTL(s) conferring the improved phenotype and for identifying further or alternative markers associated to the QTL(s). Such further markers are characterized by their location, namely close to the 48 markers disclosed in the present invention, and by their association with the phenotype of interest, revealed by the invention, namely tolerance to CGMMV.

The invention is thus also directed to a method for detecting and/or selecting C. *sativus* plants having the QTL(s) of the present invention, i.e. QTL1.2, and optionally QTL1.1 and/or QTL5, said QTL(s) conferring an improved CGMMV tolerance with respect to a corresponding plant devoid of said QTL(s), the method comprising:
- The detection of at least one of the following markers: allele A of CU-0006143, allele A of CU-0003007, allele A of CU-0001935, allele A of CU-0003410, allele G of CU-0002841, allele G of CU-0005159, allele A of CU-0003498, allele G of CU-0004638, allele A of CU-0000697, allele G of CU-0000649, allele A of CU-0003579, allele G of CU-0003759, allele G of CU-0001719 and allele A of CU-0000987 for QTL1.2 on chromosome 1, and
- Optionally the detection of at least one of the following markers: allele G of CU-0000760, allele G of CU-0005918, allele G of CU-0001201, allele C of CU-0004609, allele G of CU-0000028, allele A of CU-0003841, allele A of CU-0005952, allele G of CU-0000131, allele G of CU-0004274, allele G of CU-0002804, allele A of CU-0008736, allele G of CU-0000648, allele A of CU-0002063, allele G of CU-0008746, allele A of CU-0002751, allele A of CU-0002122, allele C of CU-0006155, allele G of CU-0001458, allele G of CU-0006165, allele G of CU-0006164, allele A of CU-0006180, allele A of CU-0000058, allele C of CU-0000848, allele G of CU-0001233, allele C of CU-0005032, allele A of CU-0004710 and allele A of CU-0006247 for QTL1.1 on chromosome 1, and/or
- Optionally the detection of at least one of the following markers: allele G of CU-0000533, allele G of CU-0001575, allele A of CU-0009561, allele G of CU-0007750, allele A of CU-0002973, allele G of CU-0001079 and allele A CU-0005413 for QTL5 on chromosome 5,
   in a genetic material sample of the plant to be identified and/or selected.

The method is particularly adapted in a breeding program with CUC58.4 (NCIMB accession number 43844), as initial parent, or progeny thereof, comprising the three QTLs of the invention conferring CGMMV tolerance, wherein the detection and/or selection is preferably made on conditions comprising infestation by CGMMV.

The invention also relates to a method for detecting and/or selecting *C. sativus* plants having the three QTLs of the present invention conferring the improved phenotype, on the basis of the allele detection of at least one SNP of the 27 SNPs of QTL1.1 and at least one of the 14 SNPs of QTL1.2 and at least one of the 7 SNPs of QTL5. A plant will be selected for the presence of QTL1.1 if at least one, preferably at least 2, 3, 5, 8, 10 or the 29 SNPs of QTL1.1 have the allele representative of the GMMV tolerance. A plant will be selected for the presence of QTL1.2 if at least one, preferably at least 2, 3, 5, 8, 10, 12 or the 15 SNPs of QTL1.2 have the allele representative of the CGMMV tolerance. A plant will be selected for the presence of QTL5 if at least one, preferably at least 2, 3, 5 or the 7 SNPs of QTL5 have the allele representative of the CGMMV tolerance.

The invention is further directed to a method for detecting and/or selecting *C. sativus* plants, especially *C. sativus var sativus,* inter alia cultivated cucumbers, having QTL1.2, and optionally QTL1.1 and/or QTL5, of the present invention conferring the improved phenotype, on the basis of the detection of any molecular marker revealing the presence of said QTL(s). Indeed, now that the QTL(s) of the present invention have been identified by the present inventors, the identification and then the use of molecular markers, in addition to the 48 SNPs of the invention could be done by the skilled artisan. The QTLs themselves may be characterized by the presence of at least one of the 48 SNPs of the invention (listed table 6 for QTL1.1, 8 for QTL1.2 and 10 for QTL5), but they may also be identified through the use of different, alternative markers. Also included in the present invention are thus methods and uses of any such molecular markers for identifying one or two or the three QTLs of the invention in a cucumber genome, wherein said QTL(s) confer(s) improved tolerance to CGMMV with respect to a corresponding plant devoid of said QTL(s), wherein said QTL(s) are independently characterized by the presence of at least one SNP listed Table 8 for QTL1.2, and preferably at least one SNP listed Table 6 for QTL1.1 and/or at least one SNP listed Table 10 for QTL5.

Are also included methods and uses of any such alternative molecular markers for identifying the QTL(s) of the invention in a cucumber genome, wherein said QTL(s) confers tolerance to CGMMV, wherein QTL1.2 is characterized by the presence of at least one of the 14 tolerance alleles of the 14 SNPs of table 8, and preferably, wherein (i) QTL1.1 is characterized by the presence of at least one of the 27 tolerance alleles of the 27 SNPs of table 6, and/or (ii) QTL5 is characterized at least one of the 7 tolerance alleles of the 7 SNPs of table 10.

The invention also concerns a method for detecting and/or selecting cucumber plants having QTL1.2, and optionally, QTL1.1 and/or QTL5, as defined previously, conferring tolerance to CGMMV, said method comprising:
a) assaying cucumber plants for the presence of at least one genetic marker genetically linked to QTL1.2, and optionally, (i) at least one genetic marker genetically linked to QTL1.1, and/or (ii) at least one genetic marker genetically linked to QTL5 involved in the tolerance of CGMMV,
b) Selecting a plant comprising the genetic marker linked to QTL1.2, and optionally QTL1.1 and/or QTL5 involved in tolerance to CGMMV, wherein said QTL1.2 is located on chromosome 1, within the chromosomal region delimited by CU-0006144 (SEQ ID NO:36) and CU-0001483 (SEQ ID NO:39), said QTL1.1 is located on chromosome 1, within the chromosomal region delimited by CU-0001918 (SEQ ID NO:1) and CU-0006297 (SEQ ID NO:8) and said QTL5 is located on chromosome 5, within the chromosomal region delimited by CU-0005234 (SEQ ID NO: 54) and CU-0007979 (SEQ ID NO: 57).

By association, or genetic association, and more specifically genetic linkage, it is to be understood that a genetic polymorphism of the marker (i.e. a specific allele of the SNP marker) and the phenotype of interest occur simultaneously, i.e. are inherited together, more often than would be expected by chance occurrence, i.e. there is a non-random association of the allele and of the genetic sequences responsible for the phenotype, as a result of their proximity on the same chromosome.

A molecular marker of the invention, either one of 48 markers disclosed above or alternative markers, are inherited with the phenotype of interest in preferably more than 90% of the meioses, preferably in more than 95%, 96%, 98% or 99% of the meioses.

The invention thus concerns the use of one or more molecular markers, for fine-mapping or identifying a QTL in the cucumber genome, said QTL participating to the improved phenotype of the invention, wherein said one or more markers is/are localized in one of the following chromosomal regions:
- in the chromosomal region delimited on chromosome 1 by CU-0001918 (SEQ ID NO:1) and CU-0006297 (SEQ ID NO:2) for QTL1.1,
- in the chromosomal region delimited on chromosome 1 by CU-0006144 (SEQ ID NO:36) and CU-0001483 (SEQ ID NO:39) for QTL1.2,
- in the chromosomal region delimited on chromosome 5 by CU-0005234 (SEQ ID NO:54) and CU-0007979 (SEQ ID NO:57) for QTL5,
- at less than 2 megabase units from the locus of one of the 48 SNP markers of the invention.

According to a preferred embodiment, said alternative markers are in the chromosomal region delimited by CU-0004565 (SEQ ID NO:2) and CU-0004683 (SEQ ID NO:7), preferably by CU-0001726 (SEQ ID NO:3) and CU-0001398 (SEQ ID NO:6), more preferably by CU-0001212 (SEQ ID NO:4) and CU-0001683 (SEQ ID NO:5), on chromosome 1 for QTL1.1, or by CU-0008776 (SEQ ID NO:37) and SNP CU-0000049 (SEQ ID NO:38) on chromosome 1 for QTL1.2, or by CU-0007682 (SEQ ID NO:55) and SNP CU-0000521 (SEQ ID NO:56) on chromosome 5 for QTL5.

Said one or more molecular marker(s) is/are moreover preferably associated, with a p-value of 0.05 or less, with at least one of the following SNP of the invention. More preferably the p-value is preferably less than 0.01.

The molecular marker is preferably a SNP marker. It is more preferably at less than 1 megabase from the locus of at least one of the 48 SNPs of the invention.

The QTLs are to be found in the deposited seeds NCIMB 43844.

The invention is also directed to the use of at least one of the 48 SNPs of the invention, associated with QTL1.1 on chromosome 1 (SEQ ID No: 11-35) and QTL1.2 on chromosome 1 (SEQ ID No: 40-53) and QTL5 on chromosome 5 (SEQ ID No 58 to 64) conferring CGMMV tolerance, for identifying alternative molecular markers associated with said QTLs, wherein said alternative molecular markers are:
- In the chromosomal region delimited on chromosome 1 by CU-0001918 (SED ID NO:1) and CU-0006297 (SEQ ID NO:2) for QTL1.1,
- In the chromosomal region delimited on chromosome 1 by CU-0006144 (SEQ IS NO:36) and CU-0001483 (SEQ ID NO:39) for QTL1.2,
- In the chromosomal region delimited on chromosome 5 by CU-0005234 (SEQ ID NO:54) and CU-0007979(SEQ ID NO:57) for QTL5, or
- at less than 2 megabase units from the locus of one of the 48 SNP markers of the invention.

According to a preferred embodiment, said alternative markers are in the chromosomal region delimited by CU-0004565 (SEQ ID NO:2) and CU-0004683 (SEQ ID NO:7), preferably by CU-0001726 (SEQ ID NO:3) and CU-0001398 (SEQ ID NO:6), more preferably by CU-0001212 (SEQ ID NO:4) and CU-0001683 (SEQ ID NO:5), on chromosome 1 for QTL1.1, or by CU-0008776 (SEQ ID NO:37) and SNP CU-0000049 (SEQ ID NO:38) on chromosome 1 for QTL1.2, or by CU-0007682 (SEQ ID NO:55) and SNP CU-0000521 (SEQ ID NO:56) on chromosome 5 for QTL5.

The alternative molecular markers are preferably associated with said QTL(s) with a p-value of 0.05 or less, preferably less than 0.01. The QTLs are to be found in the deposited seeds NCIMB 43844. The QTLs mentioned above, conferring the CGMMV tolerance according to the invention, are the QTLs present in CUC58.4 (NCIMB 43844).

Genetic association or linkage is as defined above; preferably the association or linkage is with a p- value of preferably less than 0.05, and most preferably less than 0.01 or even less. A molecular marker and the tolerance phenotype are inherited together in preferably more than 90% of the meioses, preferably more than 95%.

The molecular markers according to this aspect of the invention are most preferably SNP. They are more preferably at less than 1 megabase from the locus of at least one of the 48 SNPs of the invention, preferably at less than 0.5 megabases.

The invention also comprises a method for identifying a molecular marker associated with a QTL participating to CGMMV tolerance in cucumber, as described in the present application, comprising the steps of:
- identifying a genetic marker in the chromosomal region delimited on chromosome 1 by SNP markers CU-0001918 (SEQ ID NO:1) and CU-0006297 (SEQ ID NO:2) or in the chromosomal region delimited on chromosome 1 by CU-0006144 (SEQ ID NO:36) and CU-0001483 (SEQ ID NO:39) or in the chromosomal region delimited on chromosome 5 by CU-0005234 (SEQ ID NO:54) and CU-0007979 (SEQ ID NO:57),
- determining whether an allele or state of said molecular marker is associated with the phenotype of tolerance to CGMMV virus in a segregating population issued from a plant exhibiting tolerance to CGMMV virus, for example in a segregating population issued from a plant corresponding to the deposited seeds.

The invention is also directed to the use of one or more molecular marker(s) for identifying or selecting a cucumber plant comprising, in its genome, QTL(s) conferring CGMMV tolerance to *C. sativus* plants, in particular *C. sativus var sativus* plants, wherein said marker is localized:
- For QTL1.2 in the chromosomal region delimited on chromosome 1 by the SNPs CU-0006144 and CU-0001483, and
- Optionally for QTL1.1 in the chromosomal region delimited on chromosome 1 by the SNP markers CU-0001918 and CU-0006297 and/or
- Optionally for QTL5 in the chromosomal region delimited on chromosome 5 by the SNPs CU-0005234 and CU-0007979.

Alternatively, the said use of one or more molecular marker(s) for identifying or selecting a cucumber plant comprising, in its genome, QTL(s) conferring CGMMV tolerance to *C. sativus,* in particular *C. sativus var sativus* plants, wherein said marker is localized at less than 2 megabase unit from the locus of at least one of the 48 SNP markers of the invention; and wherein said molecular marker is associated with at least one of the SNP of the invention (i.e. Table 6, 8 and 10).

The molecular marker to be used according to this embodiment is obtainable inter alia by the method for identifying further or alternative molecular markers, as disclosed above. The molecular marker is preferably a SNP marker. They are more preferably at less than 1 megabase from the locus of at least one of the 48 SNPs of the invention.

According to still another aspect, the invention is also directed to a method for genotyping a plant, preferably a *C. sativus* plant or cucumber germplasm, for the presence of at least one genetic marker associated with tolerance to CGMMV infection, wherein the method comprises the determination or detection in the genome of the tested plant of a nucleic acid comprising at least one of the markers of the invention, or comprising at least one of the alternative molecular markers as disclosed above. Preferably, the method comprises the step of identifying in a sample of the plant to be tested specific sequences associated with tolerance to CGMMV, in nucleic acid comprising at least one of the alleles of the SNPs of the invention, as already disclosed.

The detection of a specific allele of a SNP can be carried out by any method well known to the skilled reader.

In view of the ability of the tolerant plants of the invention to restrict the damages caused by CGMMV infection, they are advantageously grown in an environment infested or likely to be infested or infected by CGMMV; in these conditions, the resistant or tolerant plants of the invention produce more marketable cucumber than susceptible plants. The invention is thus also directed to a method for improving the yield of cucumber plants in an environment infested by CGMMV comprising growing cucumber plants comprising in their genome QTL1.2 on chromosome 1, optionally combined with QTL1.1 on chromosome 1 and/or QTL5 on chromosome 5, wherein said QTL(s) is(are) as defined according to the previous aspects of the invention, and conferring to said plants tolerance to CGMMV. Preferably, at least one of those three QTLs is present homozygously. Still preferably, two QTLs are present homozygously. More preferably, all QTLs are present homozygously.

Preferably, the method comprises a first step of choosing or selecting a cucumber plant having said QTL(s) of interest. The method can also be defined as a method of increasing the productivity of a cucumber field, tunnel, greenhouse or glasshouse.

The invention is also directed to a method for reducing the loss on cucumber production in conditions of CGMMV infestation or infection, comprising growing a cucumber plant as defined above.

These methods are particularly valuable for a population of cucumber plants, either in a field, in tunnels, greenhouses or in glasshouses.

Alternatively, said methods for improving the yield or reducing the loss on cucumber production may comprise a first step of identifying cucumber plants tolerant to CGMMV and comprising in their genome QTL1.2 on chromosome 1, and optionally combined with QTL1.1 on chromosome 1 and/or QTL5 on chromosome 5, that confer to said plants, tolerance to CGMMV, and then growing said tolerant plants in an environment infested or likely to be infested by the virus.

The present invention is thus directed to a method for improving the yield of C. *sativus* plant in an environment infested by CGMMV comprising:
- identifying *C. sativus* plants tolerant to CGMMV, comprising in their genome QTL1.2, and optionally further comprising QTL1.1 and/or QTL5, wherein said QTL(s) confer(s) tolerance to CGMMV, and
- growing said resistant *C. sativus* plant in said infested environment.

By this method, the yield if the *C. sativus* plant is increased, inter alia more marketable Cucumber can be harvested, or more commercial cucumber are produced, or more seeds are obtained.

The tolerant plants of the invention are also able to restrict at least partially, the growth of CGMMV, thus limiting the infection of further plants and the propagation of the virus. Accordingly, the invention is also directed to a method of protecting a field, tunnel, greenhouse or glasshouse, or any other type of plantation, from CGMMV infestation, or of at least limiting the level of infestation by CGMMV of said field, tunnel, greenhouse or glasshouse or of limiting the spread of CGMMV in a field, tunnel, greenhouse or glasshouse, especially in glasshouse. Such a method preferably comprises the step of growing a tolerant plant of the invention, i.e. a plant comprising in its genome QTL1.2, and optionally QTL1.1 and/or QTL5, conferring to said plant, tolerance to CGMMV.

The invention also concerns the use of a plant tolerant to CGMMV for controlling CGMMV infection or infestation in a field, tunnel, greenhouse or glasshouse, or other plantation; such a plant is a plant of the invention, comprising in its genome QTL1.2, and optionally QTL1.1 and/or QTL5, as defined above. According to this use, the plants of the invention are therefore used for protecting a field, tunnel, greenhouse or glasshouse from CGMMV infestation.

The invention is further illustrated by the following figures and examples.

### FIGURES

Figure 1: Correlation between the QTLs and the phenotype of tolerance or resistance (each QTL separately), on the basis of the allele of one SNP for each QTL, namely CU-0002804 for QTL1.1, CU6-0005119 for QTL1.2, and CU0007750 for QTL5.
Figure 2: Correlation between the QTLs and the phenotype of tolerance or resistance for all QTLs in combination, on the basis of the allele of one SNP for each QTL, namely CU-0002804 for QTL1.1, CU6-0005119 for QTL1.2, and CU0007750 for QTL5.

### EXAMPLES

### Example 1: Phenotyping protocol and screening for resistant plants

Between 2013 and 2016, a trial was conducted, aiming to find CGMMV resistance leads in cucumber. After screening for potential leads, 60 genotypes were selected (Landraces). An Additional commercial hybrid (S-hybrid) and a breeding line (S-Breeding line) were tested as susceptible controls according to previous observation at the breeding program. The selected genotypes were as shown in Table 1.

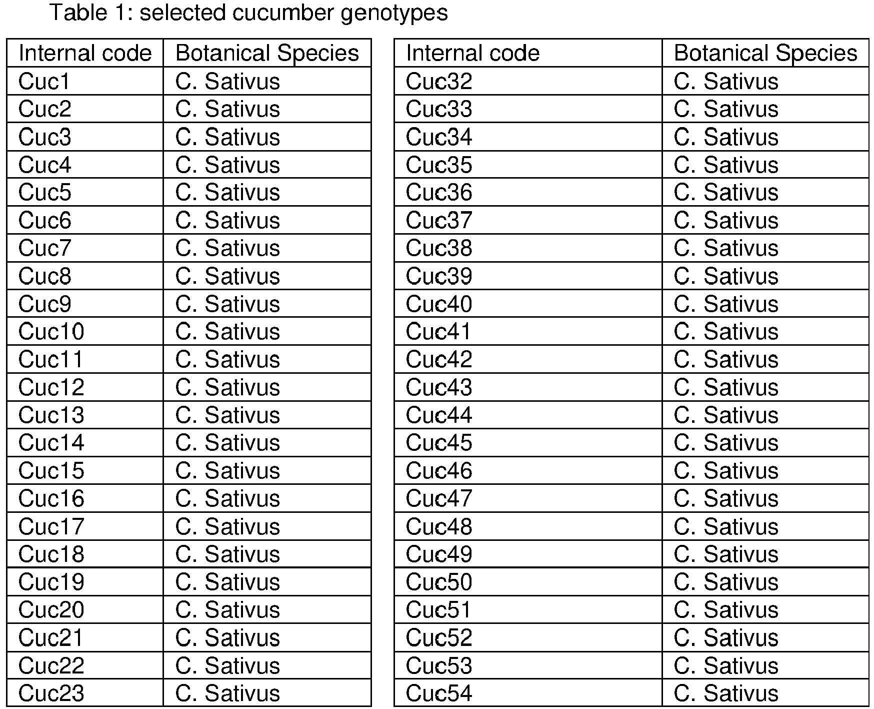

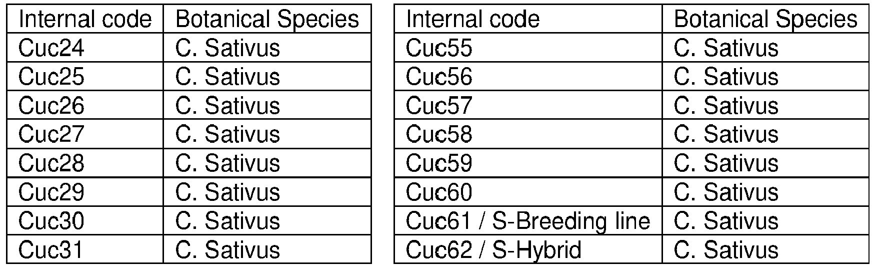

The trial was infected mechanically. The inoculation process was as follow:
The screening was done by sowing the seed in a nursery tray (between 33 to 48 seeds per line). Each plant was score separately. Plants of the trials were transplanted in the greenhouse at 21 days post infection (dpi).

Cucumber leaf containing CGMMV were used as a source of inoculum.

Inoculum preparation: Cucumber leaf contain CGMMV dried and stored in deep freeze (-80°C) until use for mechanical inoculation. In the inoculation day dry-freeze tissue were manually grind with cold water and carborundum powder. Inoculation done mechanically by rubbing plants at cotyledon stage or beginning on first true leaf development.

Propagation of the isolate done on the susceptible variety called Romi-F1.

The phenotypic evaluation was done according to four disease index:
1. Very susceptible: Strong mosaic up to the apex
2. Susceptible: Strong mosaic but not or minor on the apex
3. Intermediate: Weak mosaic but in few lower and mid leaves
4. Resistant: Completely clean or unclear spot

Evaluation on symptoms level were done 4 time during the season at, 21-, 30-, 45- and 60-days post infection (dpi). All data presented in Tables 2 to 4 correspond to evaluations made at 60 dpi. However, if plants were evaluated as very susceptible (score of 1) during the first evaluations, they were removed from the trial and definitely noted with a score of 1 (i.e., very susceptible).

**Table 2: Phenotyping evaluation during spring 2013**

| Internal Code | Phenotype | | | | Plant total number |
|---|---|---|---|---|---|
| | 4 (5) | 3 (I-R) | 2 (I-S) | 1 (I) | |
| Cuc1 | 24 | | | | 24 |
| Cuc2 | 21 | | | | 21 |
| Cuc3 | 17 | | | | 17 |
| Cuc4 | 2 | | | 4 | 6 |
| Cuc5 | | | | 4 | 4 |
| Cuc6 | | | | 15 | 15 |
| Cuc7 | | 1 | | 17 | 18 |
| Cuc8 | | 2 | 3 | 16 | 21 |
| Cuc9 | | | | 6 | 6 |
| Cuc10 | 2 | 2 | 16 | | 20 |
| Cuc11 | | 5 | | 16 | 21 |
| Cuc12 | | | | 13 | 13 |
| Cuc13 | | | | 14 | 14 |
| Cuc14 | | | | 18 | 18 |
| Cuc15 | | | | 22 | 22 |
| Cuc16 | | | | 12 | 12 |
| Cuc17 | | | | 21 | 21 |
| Cuc18 | | | 2 | 19 | 21 |
| Cuc19 | | | | 20 | 20 |
| Cuc20 | | | | 18 | 18 |
| Cuc21 | | | | 15 | 15 |
| Cuc22 | | | | 20 | 20 |
| Cuc23 | | | | 7 | 7 |
| Cuc24 | | | | 13 | 13 |
| Cuc25 | | 2 | 3 | 9 | 14 |
| Cuc26 | | 5 | 2 | 1 | 6 |
| Cuc27 | | 9 | 3 | 3 | 15 |
| Cuc28 | | | | 7 | 7 |
| Cuc29 | | | | 2 | 2 |
| Cuc30 | | | 2 | 3 | 5 |
| Cuc31 | | | 1 | 5 | 6 |
| Cuc32 | | 1 | 4 | 6 | 11 |
| Cuc33 | | | 3 | | 3 |
| Cuc34 | | 1 | 2 | 4 | 7 |
| Cuc35 | | 4 | | 10 | 14 |
| Cuc36 | | | | 10 | 10 |
| Cuc37 | | | | 14 | 14 |
| Cuc38 | | | | 2 | 2 |
| Cuc39 | 3 | 4 | | 13 | 20 |
| Cuc40 | 3 | | | 4 | 7 |
| Cuc41 | | | | 6 | 6 |
| Cuc42 | | 1 | | 11 | 12 |
| Cuc43 | | | | 19 | 19 |
| Cuc44 | | | | 20 | 20 |
| Cuc45 | | | | 9 | 9 |
| Cuc46 | | 1 | 1 | 4 | 6 |
| Cuc47 | | | | 13 | 13 |
| Cuc48 | | | 1 | 12 | 13 |
| Cuc49 | 2 | | | 11 | 13 |
| Cuc50 | 1 | 1 | | 6 | 8 |
| Cuc51 | | | | 5 | 5 |
| Cuc52 | | | | 1 | 1 |
| Cuc61 / S-Breeding line | | | | 48 | 48 |
| Cuc62/ S-Hybrid | | | | 48 | 48 |

`

**Table 3: Phenotyping evaluation during spring 2015**

| Internal Code | Phenotype | | | | Plant total number |
|---|---|---|---|---|---|
| | 4 (5) | 3 (I-R) | 2 (I-S) | 1 (I) | |
| Cuc1 | 3 | 1 | 2 | 42 | 48 |
| Cuc2 | 2 | | | 46 | 48 |
| Cuc53 | 2 | | | 46 | 48 |
| Cuc54 | 2 | | | 46 | 48 |
| Cuc55 | 1 | | | 47 | 48 |
| Cuc56 | 2 | 3 | | 43 | 48 |
| Cuc57 | | 6 | 2 | 40 | 48 |
| Cuc58 | 6 | 6 | | 36 | 44 |
| Cuc59 | | | | 48 | 48 |
| Cuc60 | 3 | 5 | 2 | 38 | 48 |
| Cuc61 / S-Breeding line | | | | 48 | 48 |
| Cuc62 / S-Hybrid | | | | 48 | 48 |

Further to this trial, two leads were found, namely Cuc58 and Cuc60. The same year in 2016, Cuc58 and Cuc60 were self-reproduced, and their progenies were evaluated in order to confirm the best lead.

**Table 4: Phenotyping evaluation during spring 2016**

| Internal Code | Phenotype | | | | Plant total number |
|---|---|---|---|---|---|
| | 4 (5) | 3 (I-R) | 2 (I-S) | 1 (I) | |
| Cuc58.1 | 25 | 9 | 6 | 8 | 48 |
| Cuc58.2 | 32 | 5 | 6 | 5 | 48 |
| Cuc58.3 | 19 | 5 | 5 | 19 | 48 |
| Cuc58.4 | 33 | 7 | 4 | 4 | 48 |
| Cuc58.5 | 12 | 23 | 7 | 6 | 48 |
| Cuc58.6 | 26 | 12 | 1 | 9 | 48 |
| Cuc60.1 | 2 | 5 | 9 | 32 | 48 |
| Cuc60.2 | 1 | | 23 | 24 | 44 |
| Cuc60.3 | | 6 | 2 | 40 | 48 |
| Cuc61 / S-Breeding line | | | | | 48 |
| Cuc62 / S-Hybrid | | | | | 48 |

Cuc58 confirms to be the best lead. Cuc58.4 plant, progeny of Cuc58, was chosen for further work.

Example 2: Creation of a F2 mapping population derived from Cuc58.4 x Cuc61.

An F2 mapping population with a susceptible breeding line as susceptible parent were created, namely Cuc58.4 (R parent) * Cuc61 (S Parent).

In order to have a more accurate phenotyping data, the mapping population was phenotyped using the following levels: 1 = very susceptible, 3= susceptible, 5=intermediate resistant (IR), 7= more resistant than IR but not resistant, 9=resistant.

Five different evaluations of the phenotype were carried out, starting from three weeks after infection (1st evaluation) to 10 weeks after infection (5nd evaluation). The first evaluation was at the stage of seedlings in the tray, the second 2nd to 5th evaluations were done after transplanting to a greenhouse at 34, 40, 53 and 67 DPI (day post inoculation), respectively.

Example 3: QTL analysis on the F2 mapping population derived from Cuc58.4 x Cuc61.

A complete QTL analysis was carried out with available SNPs.

A first selection was carried out among the potential SNPs on the basis of their allelic variations between Cuc58.4 and Cuc61. The F2 mapping population was screened with custom made Affymetrix array (set of 3,312 SNPs), covering all the chromosome.

The genotypic analysis revealed 3 QTLs linked to the resistance:
The QTL interval on the beginning of chromosome 1 is between CU-0001726 (pos. 450,655) to CU-0001233 (pos. 2,603,822), LOD= 17.

The QTL interval on the end of chromosome 1 is between CU-0006143 (pos. 23,286,654) to CU-0000987 (pos. 24,850,252), LOD=40.

The third QTL was detected on chromosome 5, is between CU-0000533 (pos. 9,205,026) to CU-0005413 (pos. 12,603,711), LOD=15.

The sequences of the SNPs on chromosomes 1 and 5 including the surrounding sequences, and the position in the cucumber genome (according to reference genome 9930 v3 (Li et al., 2019) are given in table 5 to 10. Table 11 gives the results of the QTL analysis, namely the value of Iod and Heritability values (%var). The Lod parameter is indeed an indication of the correlation to the tolerance phenotype (highest values are indicative of a highest correlation) and the Heritability value (%var) is an indication of the effect of the marker on the phenotype of tolerance to the disease (Highest values are indicative of the highest effect on the tolerance phenotype).

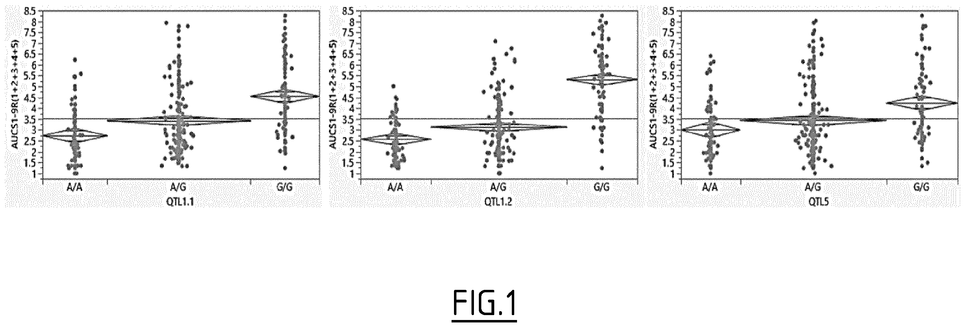

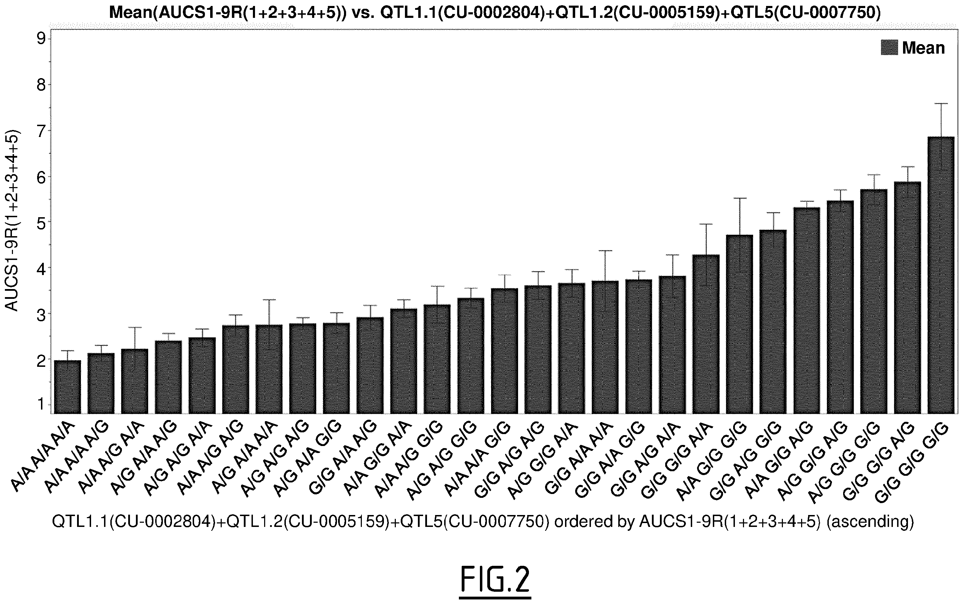

The correlation between the QTLs and the phenotype of tolerance or resistance is illustrated in FIG.1 for each QTL independently, and in FIG.2 for all QTLs in combination, and the correlation between SNPs and the tolerance or resistance are indicated in table 11. The Oneway Anova analyses of each SNP allele for each QTL are disclosed in tables 12-14. The marker linked to QTL1.2 (i.e. CU6-0005119) is explaining 42% of the resistance or tolerance phenotype observed (Rsquare 0.423506).

**Table 5: SNPs present at the borders of QTL1.1 on chromosome 1 (QTL1.1 is comprised between any combination of one SNP of Group X and one SNP of Group Y). The polymorphism is indicated within brackets. Both allele of each SNPs are representative of one boarder of QTL1.1. The position with respect to the cucumber genome as published by Li et al., 2019 (9930 v3).**

| SNP_ID | Chromo some | Position | Sequence | SEQ ID | Group |
|---|---|---|---|---|---|
| CU-000191 8 | Chr1 | 140296 | TTCTAATCCTCCTTATGAGGATCATCAT TTTCAGA[A/C]TGCTGCTTCTGGGCCTC CGCGGGAAAGTATTTTAT | 1 | X |
| CU-000456 5 | Chr1 | 287061 | GTGGTGGACCATAATGTGGGATTACAG AGCTCGTC[A/G]TTCATGGAGTAGAGAA TCTGGAAAGCGGATTCTAT | 2 | X |
| CU-000172 6 | Chr1 | 450655 | AACAAAGCCAACGTCGAAACATAATGAT TTTACTA[A/G]AGAAGAACTCATTGCCAG TAATGTAGATAGATTTA | 3 | X |
| CU-000121 2 | Chr1 | 976732 | ATAAACACCACGTCTTATCTGGAGGTAT AGTTTCA[A/G]AACTTTTTCTTTCTAAATT CATAGAAATAGATGTT | 4 | X |
| CU-000168 3 | Chr1 | 987089 | TTTACAGTATGAGCATTGGCATCAAGAA GAGTCTC[A/G]AGTGATCTCGCCTTTCG GCTCCGAACTCTACCAGC | 5 | Y |
| CU-000139 8 | Chr1 | 883294 | GAAGGCTCTCGAGAAAGGTAAGCCATG GATTAGGG[A/G]CGGATTCGGATGGTGA GGAAAATGGAGCTGTGTAC | 6 | Y |
| CU-000468 3 | Chr1 | 290685 4 | AAATGTTTGAGGTTTATCAGCCGACACA AGCTCCT[G/C]GTCATTTGGACTGAGGA CCAAATTATCATGAATCC | 7 | Y |
| CU-000629 7 | Chr1 | 313370 4 | AAAACATACGGCCATTTAGCATCTACCA CAGTAAC[A/G]CATCGTCCATCCGCTCC TATGTGTACATAGAGCCA | 8 | Y |

**Table 6: SNPs present in QTL1.1 on chromosome 1. The polymorphism is indicated within brackets. The allele representative of the presence of the QTL is in "Bold" in the bracket and reported in the last column (Tolerance T allele). The position with respect to the cucumber genome as published by Li et al., 2019 (9930 v3).**

| SNP_ID | Chromo some | Position | Sequence | SEQ ID | T Allele |
|---|---|---|---|---|---|
| CU-000076 0 | Chr1 | 955679 | ATGAAAGAGACCCCATGTTCAAATGAA GATGTCTC[A/G]TAATGGAAGAGGGAGT TCCCTCAACCAGGCAAAAA | 9 | G |
| CU-000591 8 | Chr1 | 107486 0 | TCACGTTGTAGATAAGATGCTCGACTTC GAAGATC[G/A]CCATCAATATCTCATCTT CATACTCTGCACCGTAG | 10 | G |
| CU-000120 1 | Chr1 | 113864 2 | GTTGCAGGATGTTGCTATGAAATCTATG TGAAAGC[A/G]ACCATGCGAGAAGAAGC TCGACGACAGGCAAAGAC | 11 | G |
| CU-000460 9 | Chr1 | 119356 5 | GTGCAAGAATTTCTTCACCCAACTTTAT TCTCCCG[A/C]GTAGACTTGGTAGTAGA CGAGAAGTGCTTATTCTG | 12 | C |
| CU-000002 8 | Chr1 | 119361 3 | CCACAAACTAACTGAACTAAACAGAAAA CTCACAG[A/G]ACAGAACTACTTGTGCA AGAATTTCTTCACCCAAC | 13 | G |
| CU-000384 1 | Chr1 | 118439 3 | ATATAATAAAACTGCTCATAAGAAGTAG AATATTC[A/G]TCCATTCTTTTAATTAGT TTCTCTCTCTAGATCT | 14 | A |
| CU-000595 2 | Chr1 | 121429 3 | GCTTACCACAGCCGATGCATTCCTCAT CACCGTCA[A/G]AAGAATTCGGTAGCTA CGTTTCCATTCACTGTTGA | 15 | A |
| CU-000013 1 | Chr1 | 121523 9 | CGAAACCTGGTTCCGAGTTTTATCCATT CATCGTC[A/G]CCCTCAAATTCCTCTGG GAGCTCGTATCTTTTGAG | 16 | G |
| CU-000427 4 | Chr1 | 120700 9 | GAGAGAGAGAGAGAAGAGAAAAAAGGT TGGCTATG[A/G]AAGAAGAGGGAAAATG GGGTCATCTTATAAGTGGG | 17 | G |
| CU-000280 4 | Chr1 | 150801 4 | ATGACCAAGCCATTAATCACATTAGCAC CATTCTC[A/G]GCTGCTCGATCCCGAAG ATACGCGTCCAGAACCTC | 18 | G |
| CU-000873 6 | Chr1 | 152746 3 | GCATAGGTTCATTTCTGAAGGTCCAGT GGGCTAGA[A/G]AAAAGTTGACCCACTA GATTGGGGTGGCAACTTGT | 19 | A |
| CU-000064 8 | Chr1 | 161280 4 | TGTACATGGTTTCGTGCTATGGCTGGA CTTCGAGG[A/G]GGATGCAATTTCGACG CATTTGTTCTCTGCCCAAA | 20 | G |
| CU-000206 3 | Chr1 | 161162 2 | GTTTTGGATTGCTGAGAAGGAGAATGA AGGTTTCC[A/G]CCATTTGAAGGGATTT GAAGAACTTCCATGAGAGA | 21 | A |
| CU-000874 6 | Chr1 | 162748 8 | GAAAGGGAAAGCGAGATTTATAGAGGT AAGAAAAG[G/A]AAAGAAAAAAAAGCAA AATCCCGACTAATTTCCGG | 22 | G |
| CU-000275 1 | Chr1 | 207080 7 | AGATTATAATTATTATAATAACAAAACTT GGTATT[A/G]AGAGAATCACACCTTGGA CACTTCAAAGCTTGGTG | 23 | A |
| CU-000212 2 | Chr1 | 214247 1 | AGTAACATCTTTAGAAACTGAAGGAGTG CCCTGCT[A/C]TGTTAAACACGTCGTCT TTCTTAGTATACAGATTT | 24 | A |
| CU-000615 5 | Chr1 | 207762 1 | AAGCGTCATCAAAAGGCTGAACTCATTC TGCTGCG[C/A]GACATTGCATAAATCCC CTGAGCACGACCTATCAA | 25 | C |
| CU-000145 8 | Chr1 | 213084 2 | AATCGTATCCATTTCTTCCAAACTCTTC GCCTGTA[A/G]GATATGGTCCAGTAACC AAATTTACCACCTCCTTG | 26 | G |
| CU-000616 5 | Chr1 | 220270 7 | TGGGTCGATGTTTCTTGCGTAGGATTTG GTTGCTG[G/A]GTAGAACTTACGCCAAA TGACAGCAGAATTAAACA | 27 | G |
| CU-000616 4 | Chr1 | 219156 8 | GAATGCAAAATGAACGTGACCCCGGCG AGAACCAA[G/A]AGTGTTCCAACCACTC GAGAACGGAGACCATGCTG | 28 | G |
| CU-000618 0 | Chr1 | 248581 8 | GATGGAAAGCCATTCCATTTTAGTTCAG AGAATGA[A/G]ATTTCTCCTTCTTCTATG ATTGTCCAAAAGTGTAC | 29 | A |
| CU-000005 8 | Chr1 | 249681 6 | TCAGGATGGAAGGGTATCATTTTACGCT CTCCTCG[A/G]AAACTTGTGTGAAGCTA TTCTCTATTTTTACACTC | 30 | A |
| CU-000084 8 | Chr1 | 256197 8 | GAAACTGAAGTTGCACATAAATTTATAC ACTGGTG[A/C]GTCACTGCTTCCACATC AGTTTCCAAATATACTTC | 31 | C |
| CU-000123 3 | Chr1 | 260382 2 | CTCGGTTTTGGGAAGTGTAACACTTTAA CAGCTTC[A/G]AAGCAGTATTCAGTACA CAGTAACAAAACGAAATA | 32 | G |
| CU-000503 2 | Chr1 | 255500 6 | TTCGGCGCCCTTTAGCTTCCATGATTTA GAGATAA[A/C]GAGTATTAGAAGCGAGA AGAGAAAGCTGAGTGTTG | 33 | C |
| CU-000471 0 | Chr1 | 268794 5 | ATGTTGATATACCCGTCCTTATCAGAGT CGAGATC[A/G]TCCATGACAGATCGAAG CTCTTCTAAGGGGATGGC | 34 | A |
| CU-000624 7 | Chr1 | 269566 3 | AACTCAGGCTTAGCATCAAACAAACAAA CAAGCAA[A/G]GCAAGGTGCAATACGG GGATCGATAATCAAGTAAA | 35 | A |

**Table 7: SNPs present at the borders of QTL1.2 on chromosome 1 (QTL1.2 is comprised between any combination of one SNP of Group X and one SNP of Group Y). The polymorphism is indicated within brackets. Both allele of each SNPs are representative of one boarder of QTL1.2. The position with respect to the cucumber genome as published by Li et al., 2019 (9930 v3).**

| SNP_ID | Chromos ome | Position | Sequence | SEQ ID | Group |
|---|---|---|---|---|---|
| CU-0006144 | Chr1 | 23301167 | GAGCTCAACCTTTTGATTTAGCTGCTT AGCTCTTC[A/G]TTGCACTGTTGTTAGT TTTGTTATAGCTGTTTGTG | 36 | X |
| CU-0008776 | Chr1 | 23292580 | GTGGGTATGAAGCTGTAGTTACTAAAT ATTAATGA[G/A]TCATTAAGTGAGAATT AGCGAAGATTCAGGGTTTT | 37 | X |
| CU-0000049 | Chr1 | 25333493 | AAATACAACTAAGAACTTTATTATGTAT TAAAAAT[A/G]TGTCCTCCGTCGCCGG CGGTAGCAATCTTTAATTG | 38 | Y |
| CU-0001483 | Chr1 | 25331856 | TCCCCAATTCAACTGATGGAGAAATCA TCATGAAA[A/G]TCTACCACTTGTTTTC TTTGGTGCAAAATCTTGGC | 39 | Y |

**Table 8: SNPs present in QTL1.2 on chromosome 1. The polymorphism is indicated within brackets. The allele representative of the presence of the QTL is in "Bold" in the bracket and reported in the last column (Tolerance T allele). The position with respect to the cucumber genome as published by Li et al., 2019 (9930 v3).**

| SNP_ID | Chromo some | Position | Sequence | SEQ ID | T Allele |
|---|---|---|---|---|---|
| CU-000614 3 | Chr1 | 2328665 4 | GTTTTAGTGTTGATTATTAATGTGTTGTTGTT GAT[A/G]GGGATTCTGTGGTTAACCCACAAA TCCATTTGTAA | 40 | A |
| CU-000300 7 | Chr1 | 2343617 3 | ATTGCTGAAAAAGTCCAACCTTGACCTTTC CGGG[A/C]GGGGCTTGTCCGACTGTTTGTTT TGGTGGGTACCT | 41 | A |
| CU-000193 5 | Chr1 | 2357150 3 | TAAAATGAGAGGTATCTATAATTTTATGTATA AAA[A/G]GTTGACTAAACTGAAGTTCATGCTA CCAGGGAATT | 42 | A |
| CU-000341 0 | Chr1 | 2357404 3 | AGAGAGGAAAAAGTGGGCATCCGAGCAAAG GATCT[A/G]AGGGTTCTAGGGGCGAACATTG GGTGATTCAGAAG | 43 | A |
| CU-000284 1 | Chr1 | 2387287 4 | TTCCGCTGGATATTTTCTTCTGCAGTGAAAA AATC[A/G]TCTTCTTCTTGTTTTCCAATTCCGA GTTTGGGATC | 44 | G |
| CU-000515 9 | Chr1 | 2397921 9 | TTTGATAAACCCATCYTTTACTCTTCTCCAAG TTC[A/G]TACTTCATCCTGAGTTTGAGCGAGT TCAAGCTCGC | 45 | G |
| CU-000349 8 | Chr1 | 2400396 0 | GTATCATTTTTATTATTATTAGCTTCATCGGA GGT[A/G]TTATCAATATCATTGCTTCTGGCTG GGAGTTTACT | 46 | A |
| CU-000463 8 | Chr1 | 2402386 1 | TTGCCAACCTCGATTGCATCTGCTAGGTCCT CAGT[A/G]GCATCTGCTCTCTTTGAATAGCGT TTTGCAAGTTC | 47 | G |
| CU-000069 7 | Chr1 | 2455158 6 | TGGATCATCGTCTTTGGTTGTTGCTGGAGGT GGTC[A/G]TATTGGTGTGATGGTTGCATTGTT AGGGATGTTTG | 48 | A |
| CU-000064 9 | Chr1 | 2455966 3 | TTGTTCATAGTCTTTACAACACATTAGAAGAA GAA[A/G]TTAAGGCAAGCTTCTAAGAAAGACA AAAGGAAGCT | 49 | G |
| CU-000357 9 | Chr1 | 2457932 7 | TGACTCGTAGACTGAATGCCTCCGACTGATG TAAC[A/G]ACAGATCCCGAAGTTGGACCTAC AGACATTTGGTT | 50 | A |
| CU-000375 9 | Chr1 | 2449662 0 | GATCCATGTTTTCATCTTCCATTTCCAACAAC TCC[A/G]CCACCCCCACCACCTTTCAAAACG ACAACCATCTC | 51 | G |
| CU-000171 9 | Chr1 | 2471263 2 | TATCTTCATGCTTTCTTTGATAACCTAGCTGA CAC[A/G]GACTGCGACGATAGCTCCGATGAA CCATGTCAGGA | 52 | G |
| CU-000098 7 | Chr1 | 2485025 2 | CAAGCATGCATCTATATTTCCCATGCGGAAT CATA[A/C]GAGGTGCTCTTTCAAATTTGGGAA TTCCTTGTGCG | 53 | A |

**Table 9: SNPs present at the borders of QTL5 on chromosome 1 (QTL5 is comprised between any combination of one SNP of Group X and one SNP of Group Y). The polymorphism is indicated within brackets. Both alleles of each SNPs are representative of one boarder of QTL5. The position with respect to the cucumber genome as published by Li et al., 2019 (9930 v3).**

| SNP_ID | Chrom osome | Position | Sequence | SE Q ID | Grou p |
|---|---|---|---|---|---|
| CU-0005234 | Chr5 | 6966437 | GTCGTTGGGGGACATGTTAAATCCATGGCT GTCTA[A/G]AAGGAATTTCCATATCATTGAGA TCTAGGTGCACA | 54 | X |
| CU-0007682 | Chr5 | 6958413 | TCAAGATGAGGAAAATGAAAACAGATAAGG AATCA[A/G]GATGAGGTAAGGAACTAAGAAG GAGGGTTCCTCCC | 55 | X |
| CU-0000521 | Chr5 | 13365339 | GCTCTAGATAGTGTTATTGATACAATTTATTT GAC[A/G]AAGTCAATGCAAATAGTTAGAGAA TGACAGTTAAA | 56 | Y |
| CU-0007979 | Chr5 | 13268309 | TGAAGTACCTCAAAAGCCAAATCAAGTTTTA AAAA[A/G]GGTATGCTACATAGGTAGAGACA TTTCTATATGTC | 57 | Y |

**Table 10: SNPs present in QTL5 on chromosome 5. The polymorphism is indicated within brackets. The allele representative of the presence of the QTL is in "Bold" in the bracket and reported in the last column (Tolerance T allele). The position with respect to the cucumber genome as published by Li et al., 2019 (9930 v3).**

| SNP_ID | Chromo some | Position | Sequence | SEQ ID | T Allele |
|---|---|---|---|---|---|
| CU-0000533 | Chr5 | 9205026 | CACAGTCGTCACACACAAAGTTAAAGCAGC ACACA[A/G]GTTTATATGGTTCAAAGTATGG GGGCGTGGAAGAT | 58 | G |
| CU-0001575 | Chr5 | 9280664 | TTACAGGAGATGCTCACAAGACTTGGTTTGA AGGA[A/G]TCATGATCAATTAAAGGGGCCAC CATTTTTGACCA | 59 | G |
| CU-0009561 | Chr5 | 9762568 | AAGAAAACAACTTACTTCTTCAAGTGCCCAA AAGA[A/C]CCTGAATATATGGTAAGCATGAT CTTTAAACTTTA | 60 | A |
| CU-0007750 | Chr5 | 10078304 | ACCCATTGTGTGTCCTTGTCCACATGGTGTT GCAC[A/G]CCAACTTGTCGCCTGCTAACCAC TTTTGGCAAGCC | 61 | G |
| CU-0002973 | Chr5 | 11482506 | CATGAGAAGAGAAATTTACTAGGGCGTAGG GCCAC[A/G]ACGATTAACATTCTAAAACAAG CGCGGCGACGGCG | 62 | A |
| CU-0001079 | Chr5 | 12608558 | CTCTCTYTCTTCTCTATCAATTTTACGAAGTT GCG[A/G]ACGATCAACATTCCCTCGTTTGTT ATAATACTTTC | 63 | G |
| CU-0005413 | Chr5 | 12603711 | TCTTTTTGGATTCCGGGCAGCCAAGCGAAT ATCTG[A/C]AAGAAAAGAAGTAAACCAATAAA AACATATTCAAT | 64 | A |

**Table 11: QTLs analysis of the F2 population. Correlation between SNPs and the tolerance or resistance. The position with respect to the cucumber genome as published by Li et al., 2019 (9930 v3). The Lod parameter is indeed an indication of the correlation to the tolerance phenotype (highest values are indicative of a highest correlation) and the Heritability value (%var) is an indication of the effect of the marker on the phenotype of tolerance to the disease (Highest values are indicative of the highest effect on the tolerance phenotype).**

| **SNP_id** | **CHROMOSOME (9930 v3)** | **POSITION (9930 v3)** | **Iod*** | **%var* *** |
|---|---|---|---|---|
| CU-0006144 | Chr1 | 23301167 | 0,28 | 40,55 |
| CU-0008776 | Chr1 | 23292580 | 0,28 | 40,55 |
| CU-0006143 | Chr1 | 23286654 | 35,40 | 39,00 |
| CU-0003007 | Chr1 | 23436173 | 36,90 | 41,09 |
| CU-0001935 | Chr1 | 23571503 | 37,08 | 41,28 |
| CU-0003410 | Chr1 | 23574043 | 37,08 | 41,28 |
| CU-0002841 | Chr1 | 23872874 | 40,56 | 43,11 |
| CU-0005159 | Chr1 | 23979219 | 40,33 | 43,07 |
| CU-0003498 | Chr1 | 24003960 | 41,99 | 44,40 |
| CU-0004638 | Chr1 | 24023861 | 42,16 | 44,49 |
| CU-0000697 | Chr1 | 24551586 | 39,49 | 43,10 |
| CU-0000649 | Chr1 | 24559663 | 39,63 | 43,13 |
| CU-0003579 | Chr1 | 24579327 | 39,63 | 43,13 |
| CU-0003759 | Chr1 | 24496620 | 40,55 | 43,56 |
| CU-0001719 | Chr1 | 24712632 | 37,06 | 41,41 |
| CU-0000987 | Chr1 | 24850252 | 33,24 | 39,35 |
| CU-0000049 | Chr1 | 25333493 | 0,42 | 37,53 |
| CU-0001483 | Chr1 | 25331856 | 0,46 | 37,44 |
| CU-0001918 | Chr1 | 140296 | 0,10 | 10,95 |
| CU-0004565 | Chr1 | 287061 | 0,18 | 11,61 |
| CU-0001726 | Chr1 | 450655 | 0,21 | 13,04 |
| CU-0001212 | Chr1 | 976732 | 0,11 | 12,88 |
| CU-0001683 | Chr1 | 987089 | 0,11 | 12,88 |
| CU-0001398 | Chr1 | 883294 | 0,11 | 12,88 |
| CU-0000760 | Chr1 | 955679 | 8,77 | 12,88 |
| CU-0005918 | Chr1 | 1074860 | 8,75 | 12,70 |
| CU-0001201 | Chr1 | 1138642 | 8,84 | 13,13 |
| CU-0004609 | Chr1 | 1193565 | 8,84 | 13,00 |
| CU-0000028 | Chr1 | 1193613 | 8,94 | 13,23 |
| CU-0003841 | Chr1 | 1184393 | 8,94 | 13,23 |
| CU-0005952 | Chr1 | 1214293 | 8,95 | 13,58 |
| CU-0000131 | Chr1 | 1215239 | 8,95 | 13,58 |
| CU-0004274 | Chr1 | 1207009 | 8,95 | 13,58 |
| CU-0002804 | Chr1 | 1508014 | 9,52 | 14,69 |
| CU-0008736 | Chr1 | 1527463 | 9,52 | 14,69 |
| CU-0000648 | Chr1 | 1612804 | 9,18 | 14,27 |
| CU-0002063 | Chr1 | 1611622 | 9,18 | 14,27 |
| CU-0008746 | Chr1 | 1627488 | 9,18 | 14,27 |
| CU-0002751 | Chr1 | 2070807 | 9,88 | 15,12 |
| CU-0002122 | Chr1 | 2142471 | 9,89 | 15,33 |
| CU-0006155 | Chr1 | 2077621 | 9,89 | 15,33 |
| CU-0001458 | Chr1 | 2130842 | 9,89 | 15,33 |
| CU-0006165 | Chr1 | 2202707 | 9,60 | 15,09 |
| CU-0006164 | Chr1 | 2191568 | 9,60 | 15,09 |
| CU-0006180 | Chr1 | 2485818 | 9,07 | 15,54 |
| CU-0000058 | Chr1 | 2496816 | 8,82 | 15,25 |
| CU-0000848 | Chr1 | 2561978 | 8,82 | 15,44 |
| CU-0001233 | Chr1 | 2603822 | 8,82 | 15,44 |
| CU-0005032 | Chr1 | 2555006 | 8,82 | 15,44 |
| CU-0004710 | Chr1 | 2687945 | 8,63 | 14,59 |
| CU-0006247 | Chr1 | 2695663 | 8,78 | 14,68 |
| CU-0004683 | Chr1 | 2906854 | 0,45 | 15,80 |
| CU-0006297 | Chr1 | 3133704 | 0,82 | 16,41 |
| CU-0005234 | Chr5 | 6966437 | 0,55 | 7,59 |
| CU-0007682 | Chr5 | 6958413 | 0,55 | 7,59 |
| CU-0000533 | Chr5 | 9205026 | 10,46 | 7,36 |
| CU-0001575 | Chr5 | 9280664 | 10,04 | 7,00 |
| CU-0009561 | Chr5 | 9762568 | 10,76 | 7,52 |
| CU-0007750 | Chr5 | 10078304 | 10,41 | 7,88 |
| CU-0002973 | Chr5 | 11482506 | 9,70 | 7,52 |
| CU-0001079 | Chr5 | 12608558 | 9,68 | 8,03 |
| CU-0005413 | Chr5 | 12603711 | 9,68 | 8,03 |
| CU-0000521 | Chr5 | 13365339 | 0,22 | 7,83 |
| CU-0007979 | Chr5 | 13268309 | 0,22 | 7,83 |

**Table 12: Oneway Anova for QTL1.1 by CU0002804**

| | | | | | | | |
|---|---|---|---|---|---|---|---|
| Rsquare | 0.153 | | Mean of response | | | 3.545 | |
| Adj Rsquare | 0.148 | | Observations | | | 338 | |
| Root Mean Square Error | 1.512 | | | | | | |

| **Analysis of variance** | | | | | | | |
|---|---|---|---|---|---|---|---|
| Source | DF | Sum of Squares | | Mean Square | F Ratio | | Prob>F |
| CU-0002804 | 2 | 139.114 | | 69.557 | 30.421 | | **<.0001*** |
| Error | 335 | 765.965 | | 2.286 | | | |
| C. total | 337 | 905.079 | | | | | |

| **Means for Oneway Anova** | | | | | | | |
|---|---|---|---|---|---|---|---|
| Level | Number | Mean | | Std Error | Lower 95% | | Upper 95% |
| A/A | 79 | 2.735 | | 0.170 | 2.401 | | 3.070 |
| A/G | 175 | 3.429 | | 0.114 | 3.204 | | 3.653 |
| G/G | 84 | 4.551 | | 0.164 | 4.226 | | 4.875 |

**Table 13: Oneway Anova for QTL1.2 by CU0005159**

| | | | | | | | |
|---|---|---|---|---|---|---|---|
| Rsquare | 0.423 | | Mean of response | | | 3.545 | |
| Adj Rsquare | 0.420 | | Observations | | | 338 | |
| Root Mean Square Error | 1.248 | | | | | | |

| **Analysis of variance** | | | | | | | |
|---|---|---|---|---|---|---|---|
| Source | DF | Sum of Squares | | Mean Square | F Ratio | | Prob>F |
| CU-0005159 | 2 | 383.306 | | 191.653 | **123.049** | | **<.0001*** |
| Error | 335 | 521.773 | | 1.558 | | | |
| C. total | 337 | 905.079 | | | | | |

| **Means for Oneway Anova** | | | | | | | |
|---|---|---|---|---|---|---|---|
| Level | Number | Mean | | Std Error | Lower 95% | | Upper 95% |
| A/A | 86 | 2.569 | | 0.134 | 2.304 | | 2.834 |
| A/G | 166 | 3.129 | | 0.096 | 2.939 | | 3.320 |
| G/G | 86 | 5.325 | | 0.134 | 5.061 | | 5.590 |

**Table 14: Oneway Anova for QTL5 by CU0007750**

| | | | | | | | |
|---|---|---|---|---|---|---|---|
| Rsquare | 0.072 | | Mean of response | | | 1.583 | |
| Adj Rsquare | 0.066 | | Observation | | | 3.550 | |
| Root Mean Square Error | 1.583 | | | | | | |

| **Analysis of variance** | | | | | | | |
|---|---|---|---|---|---|---|---|
| Source | DF | Sum of Squares | | Mean Square | F Ratio | | Prob>F |
| CU-0007750 | 2 | 65.305 | | 32.653 | 13.027 | | **<.0001*** |
| Error | 334 | 837.184 | | 2.506 | | | |
| C. total | 336 | 902.490 | | | | | |

| **Means for Oneway Anova** | | | | | | | |
|---|---|---|---|---|---|---|---|
| Level | Number | Mean | | Std Error | Lower 95% | | Upper 95% |
| A/A | 72 | 3.001 | | 0.186 | 2.634 | | 3.368 |
| A/G | 178 | 3.436 | | 0.118 | 3.202 | | 3.669 |
| G/G | 87 | 4.239 | | 0.169 | 3.905 | | 4.573 |

## Claims

1. A *Cucumis Sativus* plant tolerant to Cucumber green mottle mosaic virus (CGMMV) comprising in its genome a quantitative trait locus (QTL) on chromosome 1, wherein said QTL confers to the plant a tolerance to CGMMV.

2. The plant according to claim 1, wherein the said QTL on chromosome 1 is QTL1.2 located within the chromosomal region delimited by CU-0006144 (SEQ ID NO:36) and CU-0001483 (SEQ ID NO:39).

3. The plant according to claim 1 or 2, wherein QTL1.2 comprises at least one of the following SNP markers: CU-0006143 (SEQ ID NO:40), CU-0003007 (SEQ ID NO:41), CU-0001935 (SEQ ID NO:42), CU-0003410 (SEQ ID NO:43), CU-0002841 (SEQ ID NO:44), CU-0005159 (SEQ ID NO:45), CU-0003498 (SEQ ID NO:46), CU-0004638 (SEQ ID NO:47), CU-0000697 (SEQ ID NO:48), CU-0000649 (SEQ ID NO:49), CU-0003579 (SEQ ID NO:50), CU-0003759 (SEQ ID NO:51), CU-0001719 (SEQ ID NO:52) and CU-0000987 (SEQ ID NO:53).

4. The plant according to any one of claims 1 to 3, further comprising at least one additional QTL imparting tolerance to CGMMV, wherein said at least one additional QTL is:
- QTL1.1 located on chromosome 1, within the chromosomal region delimited by CU-0001918 (SEQ ID NO:1) and CU-0006297 (SEQ ID NO:8), and/or
- QTL5 located on Chromosome 5, within the chromosomal region delimited by CU-00005234 (SEQ ID NO: 54) and CU-0007979 (SEQ ID NO:57).

5. The plant according to claim 4, wherein:
- said QTL1.1 comprises at least one of the following SNP markers: CU-000760 (SEQ ID NO:9), CU-0005918 (SEQ ID NO:10), CU-0001201 (SEQ ID NO:11), CU-0004609 (SEQ ID NO:12), CU-0000028 (SEQ ID NO:13), CU-0003841 (SEQ ID NO:14), CU-0005952 (SEQ ID NO:15), CU-0000131 (SEQ ID NO:16), CU-0004274 (SEQ ID NO:17), CU-0002804 (SEQ ID NO:18), CU-0008736 (SEQ ID NO:19), CU-0000648 (SEQ ID NO:20), CU-0002063 (SEQ ID NO:21), CU-0008746 (SEQ ID NO:22), CU-0002751 (SEQ ID NO:23), CU-0002122 (SEQ ID NO:24), CU-0006155 (SEQ ID NO:25), CU-0001458 (SEQ ID NO:26), CU-0006165 (SEQ ID NO:27), CU-0006164 (SEQ ID NO:28), CU-0006180 (SEQ ID NO:29), CU-0000058 (SEQ ID NO:30), CU-0000848 (SEQ ID NO:31), CU-0001233 (SEQ ID NO:32), CU-0005032 (SEQ ID NO:33), CU-0004710 (SEQ ID NO:34) and CU-0006247 (SEQ ID NO:35), and/or
- said QTL5 comprises at least one of the following SNP markers: CU-0000533 (SEQ ID NO:58), CU-0001575 (SEQ ID NO:59), CU-0009561 (SEQ ID NO:60), CU-0007750 (SEQ ID NO:61), CU-0002973 (SEQ ID NO:62), CU-0001079 (SEQ ID NO:63) and CU-0005413 (SEQ ID NO:64).

6. The plant according to any one of claims 1 to 5, wherein said QTL1.2, QTL1.1 and QTL5 conferring tolerance to CGMMV are obtainable from a donor plant having the genetic background of the genome of the seeds of the plant CUC58.4,or in the progeny thereof comprising said QTL, deposited at the NCIMB under accession number 43844.

7. The plant according to any one of claims 1 to 5, wherein said plant is a progeny of, or derived from, the plant CUC58.4, seeds thereof have been deposited at the NCIMB, under accession number 43844.

8. A cell of a *Cucumis sativus* plant according to any one of claims 1 to 7, comprising in its genome QTL1.2, and optionally QTL1.1 and/or QTL5, wherein said QTL(s) confer(s) a tolerance to CGMMV.

9. A plant part of a *Cucumis sativus* plant according to any of claims 1 to 8 comprising in its genome the QTL1.2 and optionally, QTL1.1 and/or QTL5, in particular seeds, explants, reproductive material, scion, cutting, fruit, root, rootstock, pollen, ovule, embryo, protoplast, leaf, anther, stem, petiole or flowers,

10. A seed of *Cucumis sativus* plant, which is capable of developing into a plant according to anyone of claims 1 to 7.

11. A tissue culture of regenerable cells of a *Cucumis sativus* plant according any one of claims 1 to 7, wherein the regenerable cells are derived from embryos, protoplasts, meristematic cells, callus, pollen, leaves, anthers, stems, petioles, roots, root tips, seeds, flowers, cotyledons, and/or hypocotyls, and contain QTL1.2, and optionally, QTL1.1 and/or QTL5, said QTL(s) conferring a tolerance to CGMMV.

12. A hybrid cucumber plant that is tolerant to CGMMV, obtainable by crossing a cucumber plant with a CGMMV tolerant *Cucumis sativus* plant according to any of claims 1 to 7.

13. Use of a plant or seed of *Cucumis Sativus,* deposited at the NCIMB under the accession number 43844, or progeny thereof, bearing homozygously the QTL1.1, QTL1.2 and QTL5 conferring tolerance to CGMMV as a breeding partner in a breeding program for conferring tolerance to CGMMV to Cucumis sativus plants, preferably to Cucumis sativus plants that are susceptible to CGMMV.

14. A method for detecting and/or selecting a *Cucumis Sativus* plant comprising QTL(s) as defined in claim 1 to 7, conferring tolerance to CGMMV,wherein the method comprised:
- detecting said QTL1.2 by the presence of at least one of the following alleles: allele A of CU-0006143 (SEQ ID NO:40), allele A of CU-0003007 (SEQ ID NO:41), allele A of CU-0001935 (SEQ ID NO:42), allele A of CU-0003410 (SEQ ID NO:43), allele G of CU-0002841 (SEQ ID NO:44), allele G of CU-0005159 (SEQ ID NO:45), allele A of CU-0003498 (SEQ ID NO:46), allele G of CU-0004638 (SEQ ID NO:47), allele A of CU-0000697 (SEQ ID NO:48), allele G of CU-0000649 (SEQ ID NO:49), allele A of CU-0003579 (SEQ ID NO:50), allele G of CU-0003759 (SEQ ID NO:51), allele G of CU-0001719 (SEQ ID NO:52), allele A of CU-0000987 (SEQ ID NO:53), and
- optionally, detecting said QTL1.1 by the presence of at least one of the following alleles: allele G of CU-000760 (SEQ ID NO:9), allele G of CU-0005918 (SEQ ID NO:10), allele G of CU-0001201 (SEQ ID NO:11), allele C of CU-0004609 (SEQ ID NO:12), allele G of CU-0000028 (SEQ ID NO:13), allele A of CU-0003841 (SEQ ID NO:14), allele A of CU-0005952 (SEQ ID NO:15), allele G of CU-0000131 (SEQ ID NO:16), allele G of CU-0004274 (SEQ ID NO:17), allele G of CU-0002804 (SEQ ID NO:18), allele A of CU-0008736 (SEQ ID NO:19), allele G of CU-0000648 (SEQ ID NO:20), allele A of CU-0002063 (SEQ ID NO:21), allele G of CU-0008746 (SEQ ID NO:22), allele A of CU-0002751 (SEQ ID NO:23), allele A of CU-0002122 (SEQ ID NO:24), allele C of CU-0006155 (SEQ ID NO:25), allele G of CU-0001458 (SEQ ID NO:26), allele G of CU-0006165 (SEQ ID NO:27), allele G of CU-0006164 (SEQ ID NO:28), allele A of CU-0006180 (SEQ ID NO:29), allele A of CU-0000058 (SEQ ID NO:30), allele C of CU-0000848 (SEQ ID NO:31), allele G of CU-0001233 (SEQ ID NO:32), allele C of CU-0005032 (SEQ ID NO:33), allele A of CU-0004710 (SEQ ID NO:34) and allele A of CU-0006247 (SEQ ID NO:35), and
- optionally, detecting said QTL5 by the presence of at least one of the following alleles: allele G of CU-0000533 (SEQ ID NO:58), allele G of CU-0001575 (SEQ ID NO:59), allele A of CU-0009561 (SEQ ID NO:60), allele G of CU-0007750 (SEQ ID NO:61), allele A of CU-0002973 (SEQ ID NO:62), allele G of CU-0001079 (SEQ ID NO:63), allele A of CU-0005413 (SEQ ID NO:64),
in the genetic material sample of the *Cucumis Sativus* plant.

15. A method for detecting and/or selecting cucumber plants comprising QTL(s) as defined in claim 1 to 7, conferring a tolerance to CGMMV, said method comprising:
a) assaying cucumber plants for the presence of at least one genetic marker genetically linked to QTL1.2, and optionally, (i) for the presence of at least one genetic marker genetically linked to QTL1;1, and/or (ii) at least one genetic marker genetically linked to QTL5,
b) selecting a plant comprising the genetic markers and the QTL(s) conferring tolerance to CGMMV,
wherein said QTL1.1 is located on chromosome 1, within the chromosomal region delimited by CU-0001918 (SEQ ID NO:1) and CU-0006297 (SEQ ID NO:8), said QTL1.2 is located on chromosome 1, within the chromosomal region delimited by CU-0006144 (SEQ ID NO:36) and CU-0001483 (SEQ ID NO:39) and said QTL5 is located on chromosome 5, within the chromosomal region delimited by CU-00005234 (SEQ ID NO: 54) and CU-0007979 (SEQ ID NO: 57).

16. A method for reducing the loss of cucumber production in condition of CGMMV infection, comprising growing a cucumber plant comprising in its genome the QTL(s) as defined in claim 1 to 6 and 14, conferring to said plant tolerance to CGMMV, wherein said QTL(s) is present in the genome of a plant of the seeds CUC58.4, NCIMB number 43844.

17. Use of a cucumber plant tolerant to CGMMV for controlling CGMMV infestation of a field, tunnel, greenhouse or glasshouse, wherein said cucumber plant comprises in its genome the QTL(s) as defined in claim 1 to 6 and 14, conferring to said plant tolerance to CGMMV, wherein said QTL(s) is present in the genome of a plant of the seeds CUC58.4, NCIMB accession number 43844.
